# EUROPEAN PATENT SPECIFICATION

(11) **EP 4 436 471 B1**
(45) Date of publication and mention of the grant of the patent: **30.09.2026**
(21) Application number: 22817386.0
(22) Date of filing: 23.11.2022
(51) Int. Cl.: A61B 5/08, A61B 5/091, A61B 5/00, A61B 5/113

(54) **METHODS AND SYSTEMS OF CALIBRATING RESPIRATORY MEASUREMENTS TO DETERMINE FLOW, VENTILATION AND/OR ENDOTYPES**
VERFAHREN UND SYSTEME ZUR KALIBRIERUNG VON ATEMMESSUNGEN ZUR BESTIMMUNG VON DURCHFLUSS, BELÜFTUNG UND/ODER ENDOTYPEN
PROCÉDÉS ET SYSTÈMES D'ÉTALONNAGE DE MESURES RESPIRATOIRES AFIN DE DÉTERMINER UN DÉBIT, UNE VENTILATION ET/OU DES ENDOTYPES

(30) Priority: 23.11.2021 US 202163282375 P
(43) Date of publication of application: 02.10.2024
(73) Proprietor: Nox Medical ehf., 105 Reykjavik (IS)
(72) Inventor: HÖSKULDSSON, Sveinbjörn, 105 Reykjavik (IS); FINNSSON, Eysteinn, 105 Reykjavik (IS); ÁGÚSTSSON, Jón Skírnir, 105 Reykjavik (IS)
(74) Representative: Peters, Sebastian Martinus
(86) International application number: PCT/IB2022/061348
(87) International publication number: WO 2023/095029

(56) References cited:
- JP-B2- 5 525 476
- US-A1- 2012 041 279
- US-A1- 2018 049 678

## Description

### CROSS-REFERENCE TO RELATED APPLICATIONS

This application claims the benefit of and priority to United States Provisional Patent Application Serial No. 63/282,375 filed on November 23, 2021 and entitled "Methods and Systems of Calibrating Respiratory Inductance Plethysmography Measurements to Determine Flow, Ventilation and/or Endotypes,".

### FIELD OF THE DISCLOSURE

The present disclosure relates to systems, apparatuses, and methods for performing a personalized sleep study of a subject, and particularly for calibrating respiratory inductance plethysmography (RIP) data, which is then used for endotyping.

### BACKGROUND

Obstructive sleep apnea (OSA) is characterized by transient loss of ventilation during sleep, which promotes arterial hypoxemia and ensuing sympathoexcitation, and has major consequences for daytime health. Reliable clinical estimation of ventilation is important for defining the disorder, and is also important for clinical estimation of advanced olysomnographic measurement of the underlying causes (i.e., endotypic traits). Nasal pressure has been used to semi-quantitatively estimate ventilation, but nasal pressure as a surrogate for ventilation is severely limited due to oral breathing, for example. Accordingly improved techniques are desired for clinical estimation of ventilation. Here, the inventors have developed a novel strategy using respiratory inductance plethysmography (RIP), a measurement that is insensitive to breathing route, to estimate ventilation and the OSA traits.

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

Additional features and advantages will be set forth in the description which follows, and in part will be obvious from the description, or may be learned by the practice of the teachings herein. Features and advantages of the invention may be realized and obtained by means of the instruments and combinations particularly pointed out in the appended claims. Features of the present invention will become more fully apparent from the following description and appended claims, or may be learned by the practice of the invention as set forth hereinafter.

### SUMMARY

The invention is as defined in the appended claims.

A method for determining a respiratory flow from Respiratory Inductance Plethysmography (RIP) signals. The method includes obtaining a thoracic signal that corresponds to a length of a first RIP belt arranged proximate with a thorax of a subject, and obtaining an abdomen signal that corresponds to a length of a second RIP belt arranged proximate with an abdomen of the subject. The method further includes determining a respiratory flow based on the thoracic signal and the abdomen signal, wherein the respiratory flow is determined using two or more calibrations. The two or more calibrations include a first calibration applying a first calibration coefficient that relates an amplitude of a differential change in the thoracic signal to an amplitude of a differential change in the abdomen signal. And the two or more calibrations include a second calibration that corrects for a non-linearity in the determined respiratory flow.

### BRIEF DESCRIPTION OF THE DRAWINGS

FIG. 1 shows a flow diagram of a method of endotyping sleep disorders based on calibrated Respiratory Inductance Plethysmography (RIP) data;
FIGS. 2A, 2B, and 2C illustrate an example of respiratory inductance plethysmograph (RIP) belts, 2A shows an example of the wave-shaped conductors in the belts, 2B shows the cross-sectional area of each belt, which is proportional to the measured inductance, and FIG. 2C illustrates an embodiment of an RIP belt;
FIG. 2D illustrates a model of volumes for a thorax and an abdomen;
FIG. 3 illustrates a PolySomnoGraphy (PSG) setup;
FIG. 4 illustrates a simplified PolySomnoGraphy (PSG) setup;
FIG. 5 illustrates measured RIP traces;
FIG. 6 illustrates derived RIP volumes;
FIG. 7A illustrates derived RIP traces;
FIG. 7B illustrates derived RIP flows compared to pneumotach flows;
FIG. 7C illustrates a curve fit for an overestimation correction factor (OCF);
FIG. 7D illustrates derived RIP flows corrected according to the OCF;
FIG. 8 illustrates an alternative curve fit for an overestimation correction factor (OCF);
FIG. 9 illustrates a derivation of the flow and ventilation values from RIP traces;
FIG. 10A illustrates a Continuous Positive Airway Pressure (CPAP) maneuver;
FIG. 10B illustrates the ventilatory response to changes in CPAP pressure;
FIG. 11 illustrates the eupneic ventilation;
FIG. 12 illustrates a ventilation trace and scored arousals from a simulation of a respiratory model fit as an example of OSA endotyping;
FIG. 13 illustrates a flow diagram of a process to validate RIP-based endotyping.
FIG. 14 illustrates statistics for RIP ventilation with and without applying the OCF, showing respective biases in minute ventilation with and without OCF;
FIGs. 15A and 15B illustrate ventilation error without a non-linearity correction;
FIGs. 16A and 16B illustrate ventilation error with a non-linearity correction;
FIG. 17 illustrates a comparison of experimental results comparing event depth for pneumotach and RIP flow using a fixed calibration constant;
FIG. 18 illustrates a comparison of experimental results comparing event depth for pneumotach and RIP flow using a calibration constant;
FIG. 19A illustrates a comparison of experimental results comparing arousal threshold for pneumotach and RIP-based flows, respectively;
FIG. 19B illustrates a comparison of experimental results comparing upper airway collapsibility (Vpassive) for pneumotach and RIP-based flows, respectively;
FIG. 19C illustrates a comparison of experimental results comparing loop gain for pneumotach and RIP-based flows, respectively;
FIG. 19D illustrates a comparison of experimental results comparing ventilation at the arousal threshold for pneumotach and RIP-based flows, respectively;
FIG. 19E illustrates a comparison of experimental results comparing upper airway muscle compensation for pneumotach and RIP-based flows, respectively;
FIG. 20 illustrates intra-class correlation (ICC) values for RIP-based endotyping;
FIG. 21 illustrates flow traces for pneumotach, nasal canula, and RIP flows as an example of the effects of oral ventilation; and
FIG. 22 illustrates schematic diagram for a device that performs the method illustrated in FIG. 1;
FIG. 23A illustrates a plot comparing event depth in terms of the percent ventilation for normal breathing (eupnea) for pneumotach ventilation compared to RIP-based ventilation;
FIG. 23B illustrates a plot of pneumotach ventilation compared to ventilation based on nasal cannula measurements;
FIG. 24 illustrates ventilation as a function of time exhibiting a cycle of hypoventilation (below eupnea) followed by hyperventilation (above eupnea); and
FIG. 25 illustrates a conversion of flow values to ventilation values, which exhibit periods of hypopnea and apnea.

### DETAILED DESCRIPTION OF VARIOUS EMBODIMENTS

An object of the present application is to provide methods, systems and apparatuses that increase the accuracy of estimates of ventilation based on respiratory inductance plethysmography (RIP) measurements. The accuracy of the ventilation estimates is improved through a series of calibration as described below. These calibrations include, without limitation to order, (1) determining a period during which the RIP-belts calibration is constant, (2) determining a ratio (k) between the abdominal and thoracic volumes, (3) calibrating an overestimation correction factor (OCF) for RIP paradox overestimation, and calibrating a correction for RIP non-linearity. The estimates of ventilation may then be used in predictions of endotyping and/or phenotyping based on respiratory inductance plethysmography (RIP) measurements. The endotyping and/or phenotyping are rendered more accurate by the increase in accuracy in the estimates of ventilation. As stated above, this increase in accuracy derives from improvements in methods of calibrating the respiratory inductance plethysmography (RIP) measurements, including, e.g., calibrations to determine the ratio of abdominal to thoracic volume, calibration correcting for RIP paradox overestimation, and calibration correcting for RIP non-linearity.

The RIP measurements may be obtained from Simplified Sleep Studies (SSS). SSS may serve as valuable tools for monitoring sleep or care management of individuals being treated for different medical disorders, and particularly to provide methods, systems and apparatuses with increased the accuracy. The SSS may be performed using devices applied by the patient himself without requiring nightly or regular professional assistance.

Obstructive sleep apnea (OSA) endotyping is a method for identifying the pathophysiological traits that contribute to obstructive sleep apnea (OSA). These traits may include upper airway collapsibility, upper airway muscle compensation, arousal threshold, and loop gain, as discussed in A. Wellman et al., "A method for measuring and modeling the physiological traits causing obstructive sleep apnea," Journal of Applied Physiology, vol. 110, no. 6, pp. 1627-1637, Jun. 2011.

Understanding the pathogenesis of OSA for individual patients can be used for targeted treatment. The methods described herein provide more accurate endotyping for the pathogenesis of OSA based on respiratory inductance plethysmography (RIP) measurements, and this more accurate endotyping may be used as an integral part of precision OSA diagnosis and treatment, as discussed in W. Randerath et al., "Challenges and perspectives in obstructive sleep apnoea: Report by an ad hoc working group of the Sleep Disordered Breathing Group of the European Respiratory Society and the European Sleep Research Society," Eur Respir J, vol. 52, no. 3, p. 1702616, Sep. 2018.

In certain non-limiting embodiments described herein, respiratory inductance plethysmography (RIP) calibration is applied to minute ventilation and OSA endotyping by calibrating the RIP data before applying it the method described in E. Finnsson et al., "A scalable method of determining physiological endotypes of sleep apnea from a polysomnographic sleep study," Sleep, vol. 44, no. 1, p. zsaa168, Jan. 2021.

OSA endotypes are derived from patients' breathing patterns. Previously, patients' breathing patterns were captured with flow sensors, but flow sensors have various drawbacks. For example, flow measurements via nasal canula fail to capture ventilation arising from mouth breathing, which may be significant for OSA, and pneumotach with oronasal mask, which do captures ventilation due to both nose and mouth breathing, is not readily available in clinical settings, for various reasons. Accordingly, for improved ventilation estimation and endotyping in clinical settings, other sensors are needed to better capture ventilation due to both nose and mouth breathing. The methods described herein address this issue using RIP measurements which determine the ventilation indirectly through changes in abdominal and thoracic volumes. In certain embodiments, RIP measurements are used to determine the amount of ventilation during each minute of sleep (minute ventilation), as described in S. Chokroverty, "Sleep and breathing in neuromuscular disorders," in Handbook of Clinical Neurology, vol. 99, Elsevier, 2011, pp. 1087-1108. And the minute ventilation is the basis for endotype determination.

Using minute ventilation for endotype determination has been validated in laboratory settings using pneumotach with a sealed oronasal mask, as described in A. Wellman et al., "A method for measuring and modeling the physiological traits causing obstructive sleep apnea," Journal of Applied Physiology, vol. 110, no. 6, pp. 1627-1637, Jun. 2011; and in S. A. Sands et al., "Phenotyping Pharyngeal Pathophysiology using Polysomnography in Patients with Obstructive Sleep Apnea," Am J Respir Crit Care Med, vol. 197, no. 9, pp. 1187-1197, May 2018. When used with an oronasal mask, the pneumotach measures unbiased flow and circumvents possible confounding effects of oral ventilation. Ventilation can be either nasal or oral. Polysomnographic (PSG) sleep studies that solely use a nasal flow sensor (nasal cannula) fail to measure oral ventilation. Accordingly, PSG sleep studies based on pneumotach with oronasal mask that measure airflow through both breathing route are superior. This is an important advantage, as patients with OSA spend a significantly larger proportion of the night in oronasal breathing than those without OSA, who are more likely to be nasal breathers. Despite this, the pneumotach with oronasal mask is not readily available in clinical settings which limits the clinical applications of endotyping. Therefore, the methods described herein advantageously provide endotyping based on data that is readily available in clinical settings by using RIP data for endotyping, such that the method can be applied to standard PSG recordings.

Discussed herein is experimental data demonstrating the reliability of the method of endotyping based on RIP data without using other flow data. The experimental data shows the results of applying endotyping to standard PSG studies (i.e., RIP data), by validating the performance of the methods described herein through comparisons to endotypes gathered from PSG flow sensors to endotypes from the gold standard oronasal pneumotach. In a PSG sleep study, a nasal cannula is used for measuring nasal ventilation.

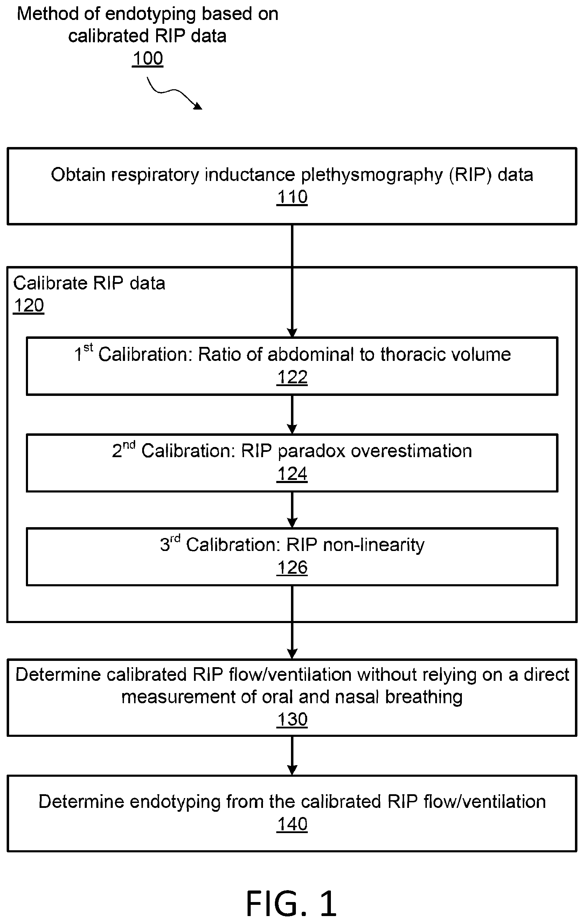

Referring now to FIG. 1, a method is illustrated for determining OSA endotypes based on the RIP data. There are several advantages to using RIP measurements, rather than nasal canula, to determine ventilation and endotyping. Even if it were feasible to use nasal cannula to detect a subset of the OSA endotypes, the nasal cannula may become dislodged during sleep, resulting in a lack of signal. Further, validating nasal cannula for the detection of OSA endotypes is challenging due to the difficulties of simultaneous pneumotach and nasal pressure measurements and the possibility of one measurement influencing the other. In contrast, pneumotach and RIP measurements are straightforward to perform simultaneously because they are respectively arranged on separate parts of the body (i.e., the head for pneumotach measurements and the chest and abdomen for RIP measurements). That is, the pneumotach/mask does not interfere with RIP measurements, so simultaneous measurement requires no adjusted setup.

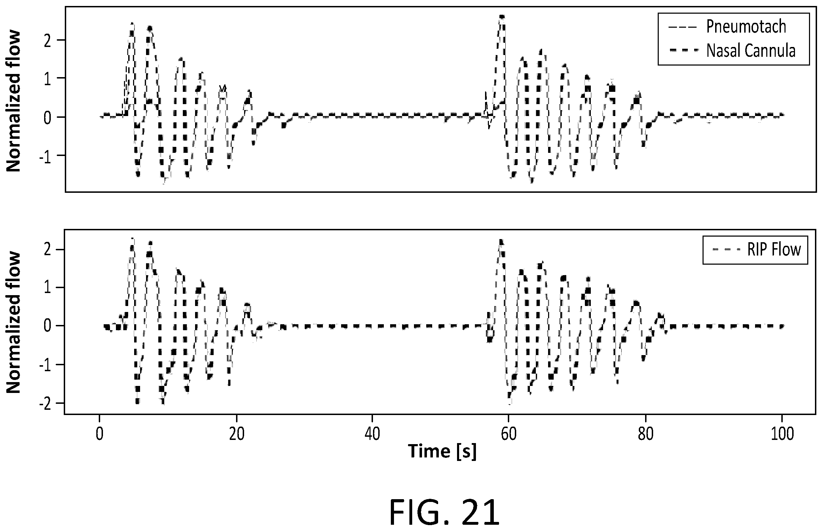

Referring now to FIG. 21 illustrates the relative advantages of using RIP measurements to predict ventilation, instead of using nasal cannula measurements. The upper plot of FIG. 21 shows the flow as a function of time for pneumotach measurements and for nasal cannula measurements. The plots show an apnea in which the flow decreases to zero for a time period and then the patient starts breathing again. During the recover breath when the patient starts breathing again a significant amount of the flow is due to oral breathing, which is not measured by the nasal cannula. In contrast, the predicted by the RIP measurements, which is shown in the lower plot of FIG. 21, closely matches the pneumotach flow, even for the recovery breathes. As can be seen, it is important to account for mouth breathing, therefore the determination of RIP flow is a significant contribution. Oral ventilation is especially important for OSA endotyping. As illustrated in FIG. 21, positive deflections are inhalations and negative deflections are exhalations. When the line flatlines, the patient has an apnea. In the upper plot, he dark grey line is measured using an oro-nasal mask while the light grey line is measured using a nasal cannula. The nasal cannula is typically used for a standard sleep study, while the pneumotach (oro-nasal) is difficult to apply at mass and therefore is not used for a standard sleep study. In the lower plot, the grey line is the RIP flow which is not sensitive to the breathing route. The methods described herein use RIP belts to provide a very accurate estimation of the oro-nasal flow.

There are several more advantages for using RIP measurements to estimate ventilation and endotyping. An airflow estimate can be derived from respiratory inductance plethysmography (RIP), which provides a surrogate measure of breathing through thoracic and abdominal movement. RIP is part of a standard PSG recording and is recommended by the American Academy of Sleep Medicine (AASM) for monitoring respiratory effort and as a secondary airflow sensor. With proper measurement and signal processing, RIP can be used to accurately and reliably measure ventilation independent of breathing route, as discussed in K. Montazeri, S. A. Jonsson, J. S. Agustsson, M. Serwatko, T. Gislason, and E. S. Arnardottir, "The design of RIP belts impacts the reliability and quality of the measured respiratory signals," Sleep Breath, vol. 25, no. 3, pp. 1535-1541, Sep. 2021. Successful validation of endotyping from RIP data has the advantage of making OSA endotyping easily available in the clinical setting. An integral step in this methodology is the calibration of the RIP signal.

Returning to FIG. 1, a non-limiting flow diagram is illustrated of a method 100 for endotyping based on calibrated RIP data. In step 110 of method 100, RIP data is obtained. In step 110, RIP belts measure an inductance from which changes in the length of the belts can be determined. Additionally, the constant (e.g., DC) signals from the belts can be used to determine the total length of the belts. The lengths of the RIP belts are related to the circumferences of the chest (Thorax) and abdomen, from which can be determined the thoracic and abdominal volumes and by extension the ventilation. However, to more accurately determine the ventilation, several calibrations are needed.

In step 120 of method 100, three calibrations are performed, although the order of these calibrations may differ in respective embodiments. In a first calibration 122, a ratio (k) is determined between the abdominal to thoracic volumes. To make this first calibration 122 more accurate a preliminary step may be taken to determine periods over which the ratio k does not change. For example, the position of the RIP belts may change throughout the night due to the patient tossing and turning in their sleep. These changes may be detected by monitoring the DC signal from the RIP belts. In a second calibration 124, a correction is made accounting for RIP paradox overestimation, as discussed below. In a third calibration 126, a correction is made accounting for RIP nonlinearity, as discussed below.

In step 130 of method 100, the above-noted celebrations are applied to the measurements from the RIP belts to generate ventilation estimates. In certain embodiments, the ventilation estimates are generated in the absence of flow measurements.

In step 140 of method 100, the ventilation estimates are used for endotyping and/or phenotyping.

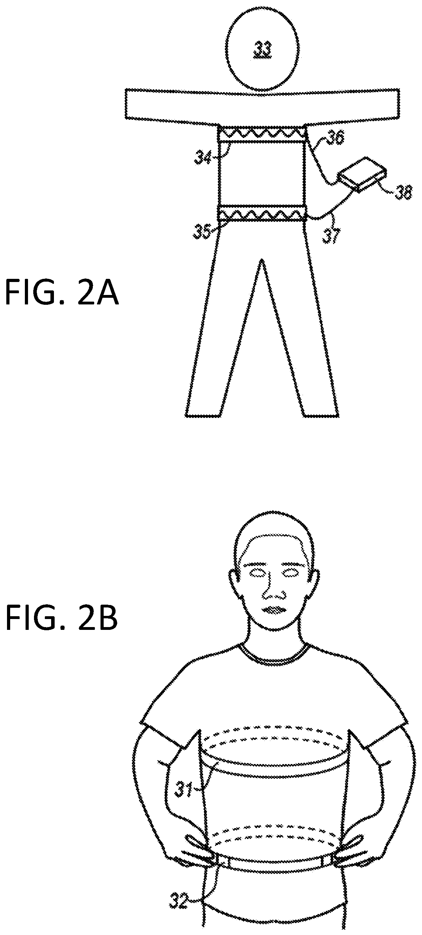

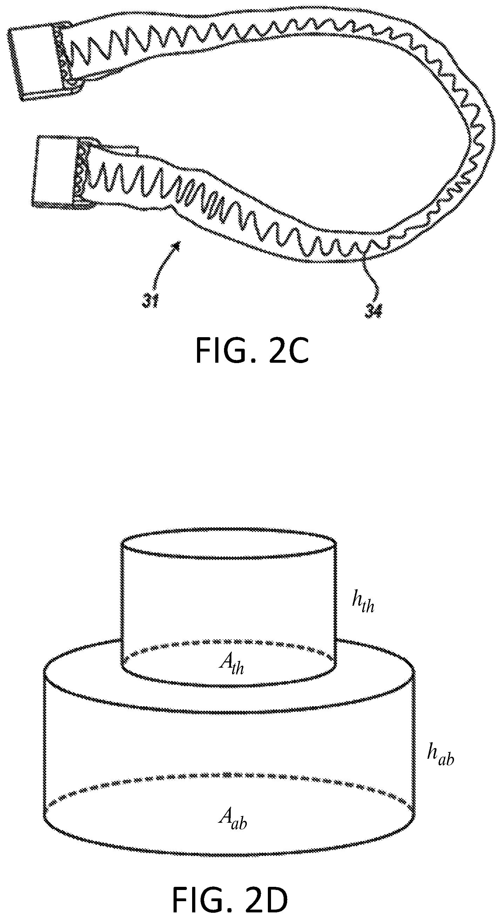

FIGs. 2A-2D illustrate an embodiment of the RIP belts. In the example of RIP, sensor belts may be capable of measuring either changes in the band stretching or the area of the body encircled by the belt when placed around a subject's body. A first belt may be placed around the thorax and second belt may be placed around the abdomen to capture respiratory movements caused by both the diaphragm and the intercostal-muscles. The area that the belt encircle can be used to derive volume and change in volume is flow $Volume = area _ abdomen * height _ abdomen + area _ thorax * height _ thorax$ $Flow = Δ area _ abdomen * height _ abdomen + Δ area _ thorax * height _ thorax$ When sensors measuring only the stretching of the belts are used, the resulting signal is a qualitative measure of the respiratory movement. This type of measurement is used, for example, for measurement of sleep disordered breathing and may distinguish between reduced respiration caused by obstruction in the upper airway (obstructive apnea), where there can be considerable respiratory movement measured, or if it is caused by reduced effort (central apnea), where reduction in flow and reduction in the belt movement occur at the same time.

Unlike the stretch-sensitive respiratory effort belts, areal sensitive respiratory effort belts provide detailed information on the actual form, shape and amplitude of the respiration taking place. If the areal changes of both the thorax and abdomen are known, by using a certain calibration technology, the continuous respiratory volume can be measured from those signals and therefore the respiratory flow can be derived.

Respiratory Inductive Plethysmography (RIP) is a method to measure respiratory related areal changes. As shown in FIGS. 2A, 2B, and 2C, in RIP, stretchable belts 31, 32 may contain a conductor 34, 35 that when put on a subject 33, form a conductive loop that creates an inductance that is directly proportional to the absolute cross-sectional area of the body part that is encircled by the loop. When such a belt is placed around the abdomen or thorax, the cross-sectional area is modulated with the respiratory movements and therefore also the inductance of the belt. Conductors 34, 35 may be connected to signal processor 38 by leads 36, 37. Processor 38 may include a memory storage. By measuring the belt inductance, a value is obtained that is modulated directly proportional with the respiratory movements. RIP technology includes therefore an inductance measurement of conductive belts that encircle the thorax and abdomen of a subject.

In another embodiment, conductors may be connected to a transmission unit that transmits respiratory signals, for example raw unprocessed respiratory signals, or semi-processed signals, from conductors to processing unit. Respiratory signals or respiratory signal data may be transmitted to the processor by hardwire, wireless, or by other means of signal transmission.

Resonance circuitry may be used for measuring the inductance and inductance change of the belt. In a resonance circuit, an inductance L and capacitance C can be connected together in parallel. With a fully charged capacitor C connected to the inductance L, the signal measured over the circuitry would swing in a damped harmonic oscillation with the following frequency: $f = 1 / \left(2π√LC\right) ,$ until the energy of the capacitor is fully lost in the circuit's electrical resistance. By adding to the circuit an inverting amplifier, the oscillation can however be maintained at a frequency close to the resonance frequency. With a known capacitance C, the inductance L can be calculated by measuring the frequency f and thereby an estimation of the cross-sectional area can be derived.

The RIP data may be obtained while performing a High-Accuracy Sleep Study (HASS), while performing a Simplified Sleep Study (SSS), or any other sleep study lying along the spectrum of sleep-study accuracy and including the acquisition of RIP belt measurements.

In certain Simplified Sleep Studies (SSS), a patient can perform the hookup himself. Simplified Sleep Studies (SSS) have been developed both as medical devices and as commercial devices made available to consumers.

In the SSS medical devices the simplification may result from simplifying the PSG sleep study by recording a subset of the PSG signals. Reducing the number of signals has however a limiting effect on the outcome. The performance and accuracy of the SSS sleep profile is reduced. SSS methods generally only work on a limited group of subjects and the accuracy and performance is especially reduced when used by those having sleep disorders. For clinical purposes, SSS are however often used for specific sleep disorders, such as to confirm sleep apnea, where reasonable accuracy can be achieved based on the measure of respiratory and oximetry parameters only, allowing the EEG, EOG, ECG and EMG signals to be skipped. Further reduced sleep studies, such as those based on oximetry only, are also practiced with further reduced accuracy and mostly used for screening purposes only.

Along the sleep study spectrum of accuracy, performance, or precision, a standard HASS may be more accurate than what may be termed a "practical" HASS, the practical HASS still providing high accuracy when compared to, for example, a Simple Sleep Study (SSS). In an embodiment, a practical HASS method (as compared to a Standard HASS or PSG) may be based on a Self Applied Somnography (SAS). SAS is a sleep study that is designed to provide close to the same information and performance for sleep profiling as standard PSG or Standard HASS but has the benefit that most people can successfully place the sensors on themselves and perform the recording. This allows the equipment and sensors to be delivered over the counter or by mail to the patient that can then perform the PSC calibration study himself before returning the SAS system to the clinic or shipping it back over mail.

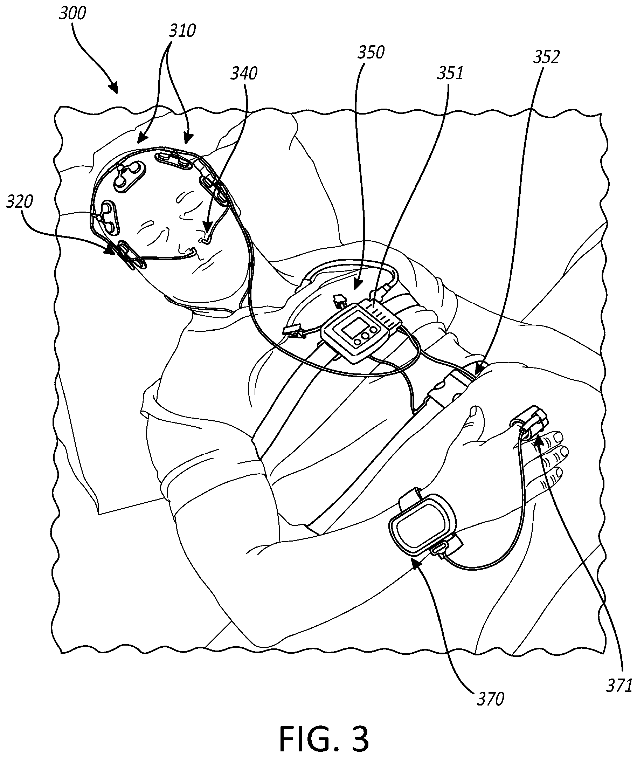

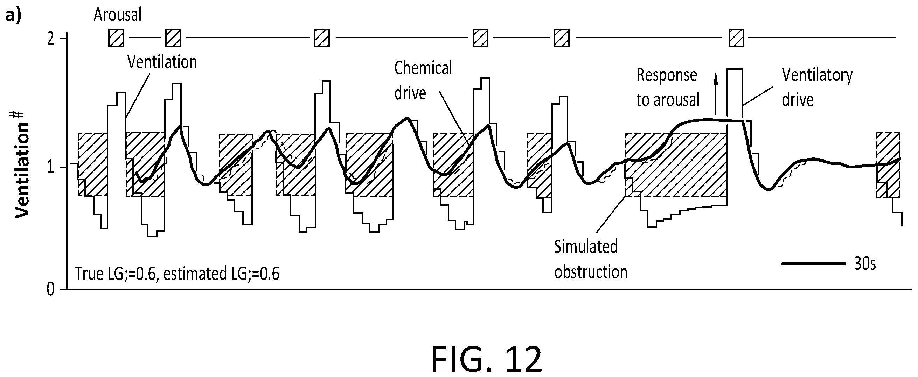

FIG. 3 shows a subject undergoing such a practical HASS according to this embodiment, in the form of a SAS. As shown in FIG. 12 a subject 300 may preferably apply the following sensors and devices to himself, or an untrained or uncertified assistant, such as a family member, roommate, or untrained or uncertified medical work. EEG electrodes 310 may be attached to the head of the subject. The electrodes 310 may be arranged in a band to ensure proper placement. The band may also include, but does not necessarily include, EOG electrodes 320 placed on one or more distal ends of the headband so as to be arranged near an eye of the subject 300. Furthermore, the patient 300 may have a nasal cannula 340 used to measure nasal breathing. The subject 300 may also have respiratory inductance plethysmography (RIP) stretchable belts 351, 352 placed around his chest (thoracic) and abdomen, respectively, to measure breathing movements. Stretchable belts 351,352 may contain a conductor (not shown) that when put on a subject 300, form a conductive loop that creates an inductance that is directly proportional to the absolute cross sectional area of the body part that is encircled by the loop. When such a belt is placed around the abdomen or thorax, the cross-sectional area is modulated with the respiratory movements and therefore also the inductance of the belt. Conductors in the belts may be connected to signal processor 350 by leads or transmitted or received by the processor 350 wirelessly. Processor 350 may include a memory storage. By measuring the belt inductance, a value is obtained that is modulated directly proportional with the respiratory movements. RIP technology includes therefore an inductance measurement of conductive belts that encircle the thorax and abdomen of a subject.

The subject 300 of the embodiment of FIG. 12 may also have a pulse oximeter 370 on the wrist and a corresponding sensor 371 on a finger, such as an index finger, to measure the blood oxygen saturation and pulse in the finger. Furthermore, the patient may also have leg EMG leads although not shown in FIG. 12.

In another embodiment, an advanced SAS system may be used that is based on the use of wireless Smart Sensors that minimize the use of cables and further simplify significantly the hookup of the sensors while maintaining the performance and high-resolution of the cabled SAS or PSG methods. Such an advanced SAS is also preferable in that movement or turning of the subject during the study is not encumbered by wires or cables.

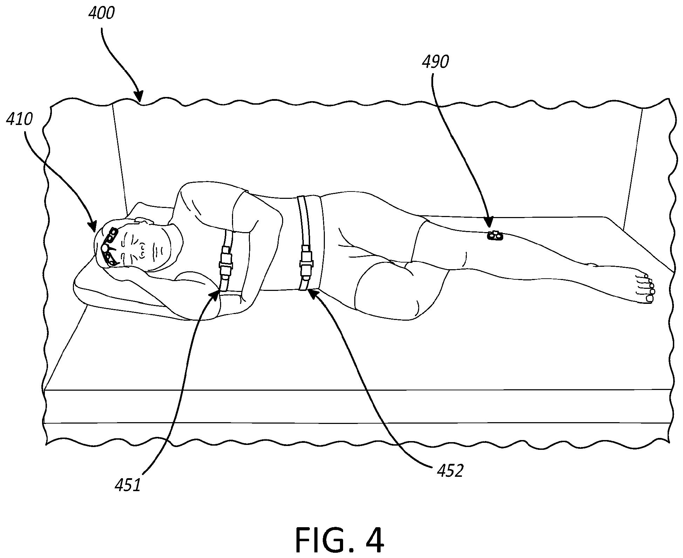

FIG. 4 shows a schematic of a subject 400 sleeping with the wireless SAS sleep study. The devices worn by the subject in such as study may include EEG electrodes 410 placed on the forehead of the subject, respiratory inductance plethysmography (RIP) belts 451, 452, and a sensor or leg EMG lead 490 applied to the leg of the subject. As noted above, these sensors and devices have been provided to the subject in such a way that the subject can easily and consistently apply the sensors and devices himself (or herself).

Returning to FIG. 1, after step 110 in which the data is acquired via the RIP belts, the RIP data is calibrated in step 120 using one or more calibrations. In a first calibration, a ratio is determined between the abdominal volume and the thoracic volume.

The first RIP calibration is intuitively understood by considering that relative changes in lung volumes can be estimated by measuring the diameter of the thorax and abdomen and adding them together in the right proportions (e.g., summing them using a proportionality constant "k"). The proportionality constant can be found by performing an isovolume breathing maneuver, inducing paradoxical movement of the abdomen and thorax, and choosing the calibration constant (k) such that the measured amplitudes of the abdominal and thoracic excursions cancel out. This is called Isovolume maneuver calibration (ISOCAL), as discussed in K. Konno and J. Mead, "Measurement of the separate volume changes of rib cage and abdomen during breathing," Journal of Applied Physiology, vol. 22, no. 3, pp. 407-422, Mar. 1967, which is incorporated herein in its entirety.

In a PSG sleep study, the calibration constant may change throughout the night. For example, the sleeping subject may move throughout the night, displacing the belts move from their original position. Consequently, when breathing movements are recorded using RIP belts, ISOCAL calibration may be challenging. To overcome this challenge, the calibration constant may be updated throughout the study by inferring the calibration constant (k) from the signals resulting from spontaneous breathing throughout the night. For example, qualitative diagnostic calibration (QDC) may be used to recalibrate the calibration constant (k) throughout the study. In certain embodiments, the calibration constant (k) is chosen such that the variance between the abdominal and thoracic tidal volumes is normalized over a moving, non-overlapping, 5 minute window.

Alternatively, the calibration constant (k) may be chosen such that the two RIP belts are simply added together in a fixed ratio of 2:1 (chest-to-abdomen).

The RIP bands measure changes in the circumference of the thorax and abdomen. Assuming a constant height of these two compartments, the RIP signals may be used to infer respective volumes (or changes in volume) for the thorax and abdomen. A flow estimate can be calculated from the RIP signals by using the time derivative of the calibrated RIP signal according to the following equation $RIP _ flow = k dRIP _ Ab + \left(1-k\right) dRIP _ Th ,$ wherein dRIP_Ab and dRIP_Th are respectively time derivatives of the abdominal and thoracic RIP signals, and k is the calibration constant.

Returning to FIG. 1, a first calibration is performed to generate the the calibration constant k, which relates the abdominal and thoracic volumes. To calibrate the belts, only information available from the RIP signals themselves is used. For example, this calibration does not depend on any (potentially unavailable) airflow signals. Advantegously, this calibration may be performed across the night in the event of overnight changes. The calibration of the the calibration constant k may be performed using the "power loss" method, which aims to find the optimal calibration constant k (where the 'calibrated' tidal volume signal = [1-*k*]RIPₜₕ + [*k*]RIP_{ab}; RIPₜₕ and RIP_{ab} are the thoracic and abdominal volume signals respectively). Optimal k is found by minimizing the ratio of the root mean square (RMS) of the 'calibrated' flow signal (derivative of the volume signal) to the sum of the RMS of the separate thoracic and abdominal flow signals, i.e.: $\frac{RMS \left(\left[1-k\right]\frac{d}{dt}{RIP}_{th}+\left[k\right]\frac{d}{dt}{RIP}_{ab}\right)}{RMS \left(\left[1-k\right]\frac{d}{dt}{RIP}_{th}\right) + RMS \left(\left[k\right]\frac{d}{dt}{RIP}_{ab}\right)}$

In principle, when the calibration constant is tuned appropriately, the paradox component seen in each separate signal ([1-*k*]RIPₜₕ, [*k*]RIP_{ab}) is maximally subtracted out in the calibrated sum. In certain embodiments, recalibration is performed when a patient moves. For example, *k* may be updated using an adaptive strategy whereby the night is subdivided into multiple *constant calibration periods* (typically >5 minutes) separated by automatically-detected movement events. The calibration constant k may be maintained constant within each period, thereby mitigating the adverse effects of non-constant tidal volume on RIP calibration.

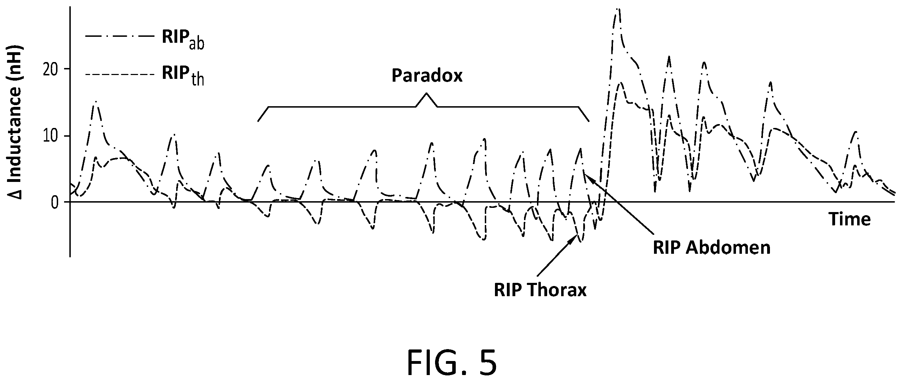

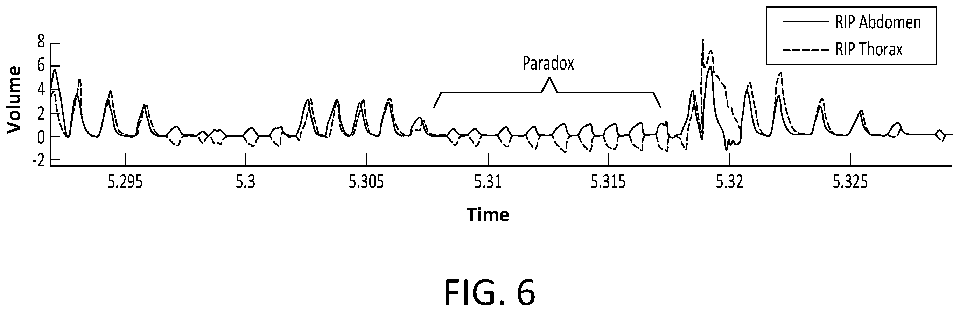

FIGs. 5 and 6 illustrate RIP measurements during an apnea. FIG. 5 illustrates the raw measurements of the inductance of the RIP belts, and FIG. 5 illustrates the volumes estimated from other measurements of the inductance of the RIP belts. The illustrated behavior is what one would expect during an apnea. The upper airway resistance increases during the apneas, as indicated by the out of phase movements of the RIP signals, which is indicated as paradox (i.e., paradoxical breathing. During this period there is no flow/ventilation and the total volume remains constant. Accordingly, like during an ISOCAL calibration, the signal during this period may be used to calibrate the calibration constant k.

The calibration constant k may change throughout a sleep study due to displacement of the RIP belts from their original locations. This can be monitored using a DC signal from the RIP belts. For example, changes to the DC signal indicate that the averaged length of the belt has changed, and a new value for the calibration constant k. Thus, the calibration constant k may take on a series of discrete values in respective constant calibration periods throughout the sleep study. Each period of constant calibration is determined by monitoring for changes in the DC signals from one or more of the RIP belts. In each constant calibration period, the calibration constant k may be determined using the above calibration process, for example, to have the volumes for the RIP abdomen and RIP thorax effectively cancel each other during the period of paradox.

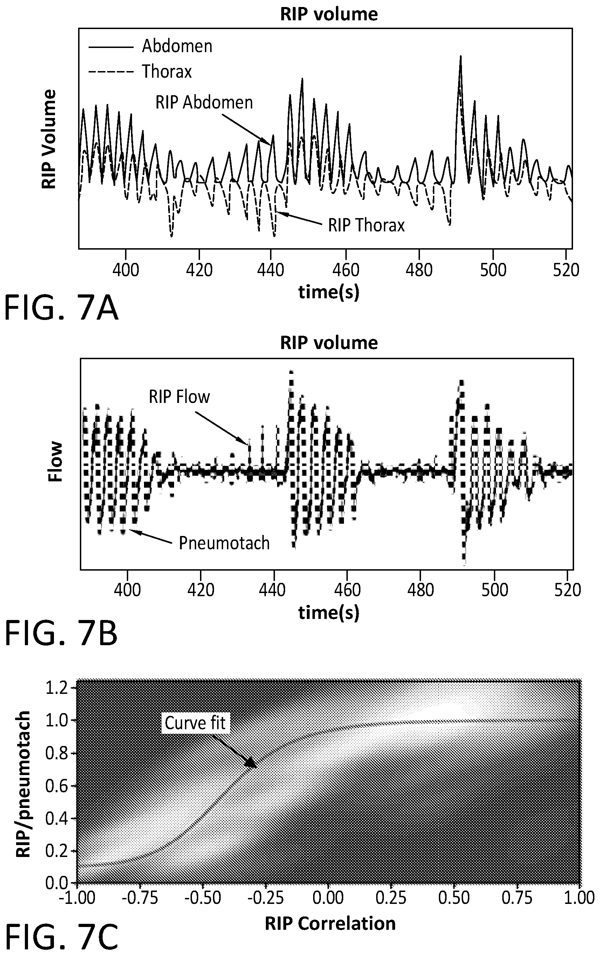

Returning to FIG. 1, a second calibration 124 is performed to determine and correct for RIP paradox overestimation. Thoracic and abdominal excursions describe just two compartments of a more complex respiratory system. Accordingly, even the optimal value of the calibration constant k does not result in the paradoxical movements being perfectly cancelled. Consequently, there is a systematic overestimation of ventilation during paradox. This overestimation is illustrated in FIGs. 7A and 7B. FIG. 7A shows the calibrated RIP volumes for the abdomen and the thorax. During paradox, these two signals are clearly out of phase (i.e., the peaks in one signal occur simultaneously with the troughs from the other). FIG. 7B illustrates (dark grey line) the RIP flow generated using the calibrated RIP volumes. Further, FIG. 7B illustrates the pneumotach flow (black line), which unlike the RIP flow goes to zero during paradox. Thus, compared to the pneumotach flow, the RIP flow overestimates the flow/ventilation during paradox.

To correct this overestimation, the amount of overestimation may be determined using the curve fit shown in FIG. 7C. In FIG. 7C, the horizontal axis is the correlation between the RIP volumes for the abdomen and thorax. As discussed above, anti-correlation (i.e., a correlation of negative one) occurs during paradox when the signals are out of phase, and during periods of normal respiration the correlation is positive. In FIG. 7C, the vertical axis is the ratio of the pneumotach flow to the RIP flow (each averaged over a short period). The whiter regions have higher concentrations of data having the corresponding x,y values for the RIP correlation and the ratio pneumotach flow/RIP flow. By minimizing an error function (e.g., the RMS error) and assuming a predefined functional form, a curve fit can be obtained. Here, positive correlations generally correspond to the curve fit value of about one, and approach a value close to zero (e.g., a value of 0.1) as the RIP volumes become anti-correlated.

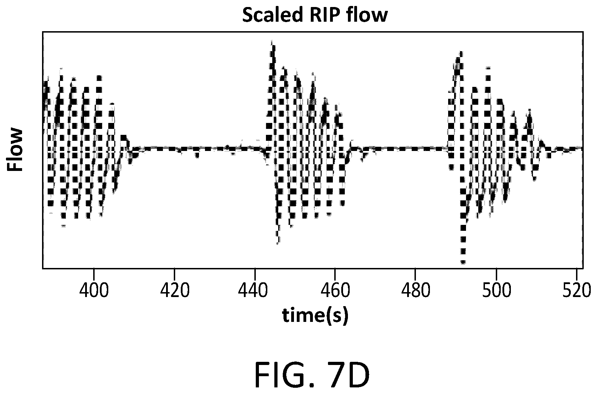

Using the above-determined curve fit, the locally averaged correlation between the RIP volumes can be calculated and input to the curve fit to calculate a multiplier (i.e., the overestimation correction factor (OCF)). This multiplier may then be multiplied by the RIP flow to mitigate the overestimation during paradox. FIG. 7D shows the RIP flow that has been scaled by the OCF. Like in FIG. 7B, FIG. 7B illustrates the scaled RIP flow as a dark grey line, and illustrates the pneumotach flow as a black line. Here, much better agreement is observed between the scaled RIP flow and the pneumotach flow than was observed in FGI. 7B between the unscaled RIP flow and the pneumotach flow.

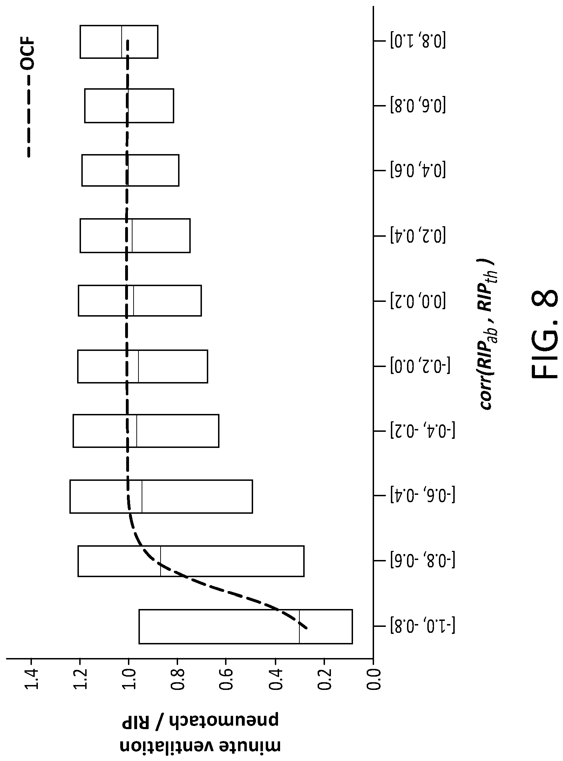

FIG. 8 illustrates another embodiment of determining the overestimation correction factor (OCF). To quantify the bias in the RIP flow during paradox and correct for it, the following analysis may be performed. First, the paradox is determined using the correlation coefficient R (Pearson) between thoracic and abdominal volume signals, for each breath. The oronasal ventilation and RIP ventilation are also calculated to examine whether the oronasal/RIP ventilation ratio was reduced with greater paradox. In FIG. 8 this relationship is plotted. More particularly, all breaths of data from multiple patients are sorted by R and binned into deciles. Next, median oronasal/RIP ventilation ratio are plotted against R. In this non-limiting embodiment, a sigmoid-shaped relationship was fit to the decile-binned data to describe the 'overestimation correction factor (OCF)'. For this exemplary data set, the curve fit is $OCF = \left(1-α\right) \left[1/\left(e-20.92\left(R-0.0441\right)+1\right)2\right] + α and α = 0.123 .$ Generally, OCF is approximately one except under severe paradox when it falls to as low as 0.12, for example. Multiplication of the calibrated RIP ventilation by OCF, on a breath-by-breath basis, may be used to obviate the systematic overestimation of ventilation during pharyngeal obstruction. Here, the OCF function is determined by using data from multiple patients, but in other embodiments, the particular values for the OCF function may be patient specific.

Returning to FIG. 1, a third calibration 126 is performed to determine and correct for RIP non-linearity. A plot of patient data with the RIP ventilation on one axis and pneumotach (e.g., oronasal) ventilation on the other axis may show a non-linear relation. For example, using RIP, ventilation may be underestimated for large breaths. Accordingly, a power-law correction factor may be applied to minimize this underestimation. In certain embodiments, the functional form of the power-law correction factor may be y=x1/β. For certain patients, it has been observed that when values of 0.75, 0.8, 0.85, 0.9 were attempted for the variable β, the optimal value was observed to be β=0.85.

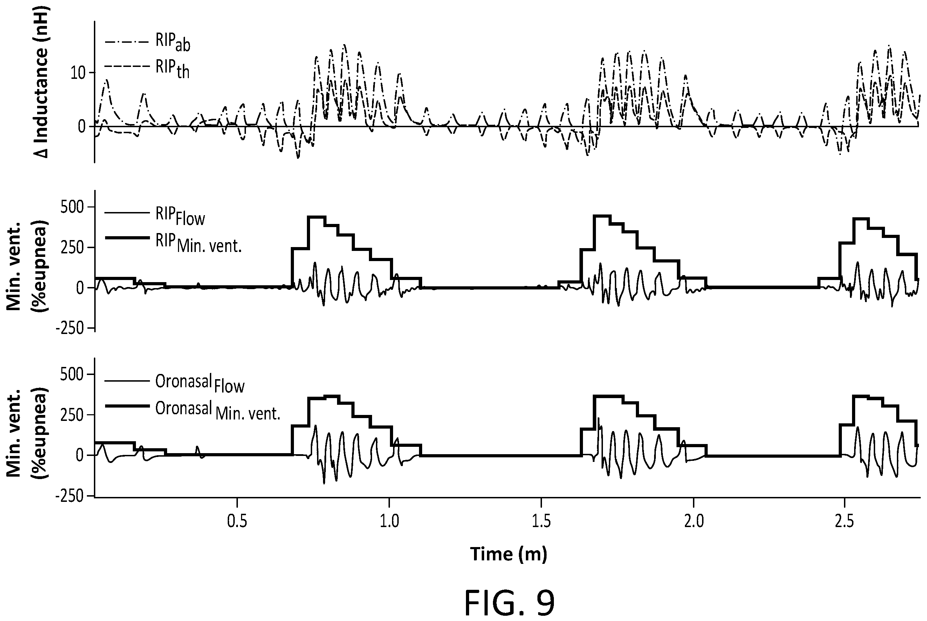

In FIG. 1, step 120 in which the calibrations are performed is followed by step 130 in which the calibrations are applied to the RIP data to generate a time series of RIP flow/ventilation values. In certain embodiments, the RIP flow/ventilation values are generated without measurements of the oral or nasal breathing. FIG. 9 illustrates starting with inductance measurements from the RIP belts, and converting these to flow values and minute ventilation values. The upper plot shows as a function of time (horizontal axis) the RIP inductance values for the abdomen and thorax. The middle plot shows the RIP flow signal derived from the time derivative of the calibrated RIP signal. Superimposed is the respective minute ventilation signal. The upper plot shows the target oronasal pneumotach signal and the target minute ventilation signal. Using the equation, $RIP _ flow = k dRIP _ Ab + \left(1-k\right) dRIP _ Th$ A flow estimate can be calculated from the RIP signals by using the time derivative of the calibrated RIP signal, where dRIP_Ab and dRIP_Th are the time derivatives of the abdominal and thoracic RIP signals, respectively and k is the calibration constant. In FIG. 9, the recorded ventilation traces of a 3-minute slice of a sleep study during a period of repeated obstructive respiratory events. In the upper plot, RIP inductance signals are shown with the Functional Residual Capacity (FRC) baseline removed to better visualize paradoxical movement during obstructive apnea periods.

Minute ventilation may be calculated by multiplying the tidal volume times the respiratory rate for breaths detected in wake and sleep. Values for the minute ventilation may be expressed as a percentage of the local average "eupneic" ventilation (7-min window). Here, "eupnea" means normal respiration, which contrast with the meaning "apea," i.e., a temporary cessation of breathing, especially during sleep.

As discussed above, the flow and minute ventilation calculated from the RIP data are improved through novel RIP calibration methods with the aim of achieving an unbiased estimate of minute ventilation from the RIP signals, compared to the gold standard oronasal pneumotach, during spontaneous breathing in sleep. For example, the calibrated RIP data may be used to determine minute ventilation and OSA endotype values that agree closely with ventilation and OSA endotype values determined using the gold standard oronasal pneumotach, validating the RIP-based method for determining flow, ventilation, and OSA endotype values. Accordingly, the methods described herein provides clinicians with the tools to make more informed decisions when diagnosing and treating patients with OSA in a standard PSG study.

Returning to FIG. 1, in step 140, endotyping is determined from the flow and/or ventilation values determined from the RIP measurements. Endotyping is useful for diagnosing and treating sleep disorders. In light of increased skepticism about using the apnea-hypopnea index as the sole indicator of OSA, endotyping can provided needed insight for treating OSA. The methods described herein improve the accessibility of OSA endotyping by developing a RIP calibration method that allows for the extraction of endotypes from the RIP signals. The validity of these methods is demonstrated by the high agreement between RIP and pneumotach derived endotypes, for all explored OSA endotypes (upper airway collapsibility, upper airway muscle compensation, arousal threshold and loop gain).

For example, the determination of the underlying cause of sleep apnea in patients has been determined by measuring loop gain, determining the level of arousability of the patient, and by measuring the collapsibility of the upper airway. Traditionally, the loop gain is measured or estimated by measuring the change in respiratory drive as a response to change in ventilation, and the arousability is determined by measuring the respiratory drive resulting in a cortical arousal of a patient.

A phenotype is the composite of an organism's observable characteristics or traits, such as its morphology, development, biochemical or physiological properties, behavior, and products of behavior. A phenotype may be a result of the genetic expression in an organism, endophenotype. Phenotyping a subjects respiratory effort may reveal information about the mechanics of the respiratory system, the response of the system to a disturbance such as reduced ventilation, the tendency of the airway to collapse, the ability of dilator muscles in the system to maintain an open airway, the internal pressures and forces acting on parts of the respiratory system or in its entirety, the recruitment of respiratory muscles, and any other function resulting in breathing or caused by breathing.

An endotype is a subtype of a condition, which is defined by a distinct functional or pathobiological mechanism. This is distinct from a phenotype, which is any observable characteristic or trait of a disease, such as development, biochemical or physiological properties without any implication of a mechanism. It is envisaged that patients with a specific endotype present themselves within phenotypic clusters of diseases.

OSA endotyping is a method for identifying the pathophysiological traits that contribute to OSA. For example, these traits may include upper airway collapsibility, upper airway muscle compensation, arousal threshold and loop gain. Understanding the pathogenesis of OSA for individual patients can be used for targeted treatment. Endotyping may be an integral part of precision OSA diagnosis and treatment.

OSA endotypes are derived from patients' breathing patterns, which are generally captured with flow sensors. The main variable of interest is the amount of ventilation during each minute of sleep (minute ventilation), which may be the basis for endotype determination. Calculating endotypes is validated in laboratory settings using the gold standard pneumotach with a sealed oronasal mask. When used with an oronasal mask, the pneumotach measures unbiased flow and circumvents possible confounding effects of oral ventilation. The methods described here take the additional step to scaling the pneumotach method to RIP results, which are available in Polysomnographic (PSG) sleep studies such that endotyping can be applied to standard PSG recordings.

As discussed below with reference to an experimental study, when applying endotyping to standard PSG studies, it is important to validate its performance by comparing endotypes gathered from PSG flow sensors to endotypes from the gold standard oronasal pneumotach. The ideal comparison is within the same study by paired intra-patient comparisons.

When applying endotyping to standard PSG studies, it is necessary to validate its performance by comparing endotypes gathered from PSG flow sensors to endotypes from the gold standard oronasal pneumotach. The ideal comparison is within the same study by paired intra-patient comparisons. In a standard PSG sleep study, a nasal cannula is used for measuring nasal ventilation. The feasibility of using the nasal cannula to detect a subset of the OSA endotypes, namely: upper airway collapsibility (Vpassive) and upper airway muscle compensation (Vcomp) as well as ventilation at the arousal threshold (Vactive) [Sands et al. 2018], has been verified on a small cohort [PUPpy, Sands2018]. However, the nasal cannula may become dislodged during sleep, resulting in a lack of signal [Guðnadóttir et al., 2019, Altman et al., 2012]. Further, validating this method is challenging due to the difficulties of simultaneous pneumotach and nasal pressure measurements and the possibility of one measurement influencing the other.

Even though the disclosure primarily discusses estimating endotypes, the methods described herein can use the RIP flow and/or RIP ventilation to estimate either phenotypes or endotypes.

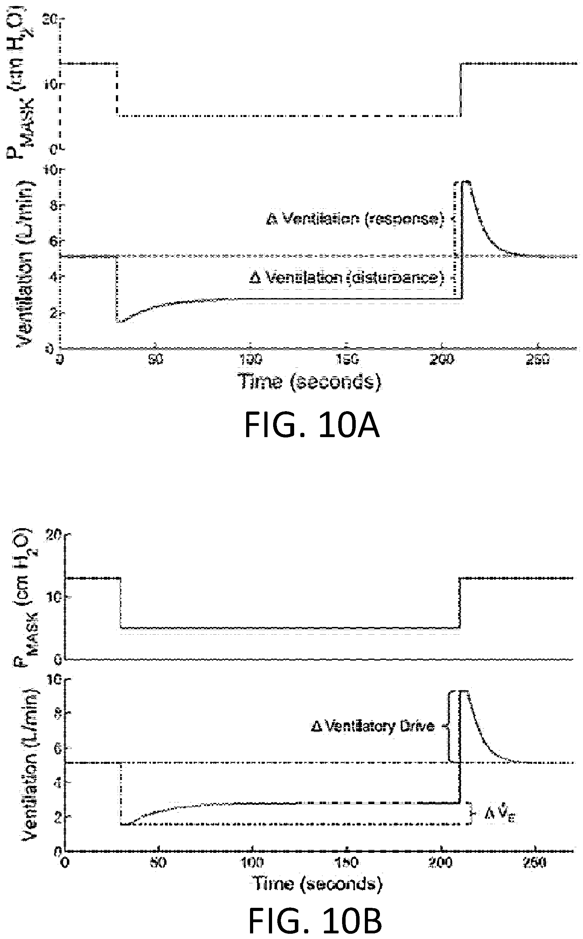

FIG. 10A illustrates a known Continuous Positive Airway Pressure (CPAP) maneuver and the resulting ventilatory response used in a endotyping protocol. The endotyping protocol involves having a patient on a CPAP at a therapeutic level. The CPAP level is lowered in an instant and kept at a lower than therapeutic pressure for a period of 3 minutes. The CPAP level is then set to the original therapeutic level. During this maneuver, the patient ventilation is measured. When the CPAP is at a therapeutic level the patient has unobstructed breaths, which are used as a reference for the later breaths. Once the CPAP is turned down ventilation is reduced. By increasing ventilatory drive, the patient manages to increase ventilation to a new steady state level. The difference between the ventilation during therapeutic CPAP and the ventilation at the new steady state is the ventilation disturbance. Once the CPAP is turned back on the airway opens up and the ventilation equals the ventilatory drive. The difference between the ventilation after turning the CPAP up and the normal unobstructed ventilation is the ventilatory response. The loop gain, LG, is found by the equation: $LG = \frac{Disturbance}{Response}$

The upper airway gain, UAG, is found by measuring how much the change in ventilatory drive changes ventilation as shown in FIG. 10B. FIG. 10B shows the ventilatory response to changes in CPAP pressure. The increase in ventilation due to increased ventilatory drive is labeled as Δ*V̇_{E}.* The UAG is calculated by the equation: $UAG = \frac{Δ {\dot{V}}_{E}}{Δ Drιve}$

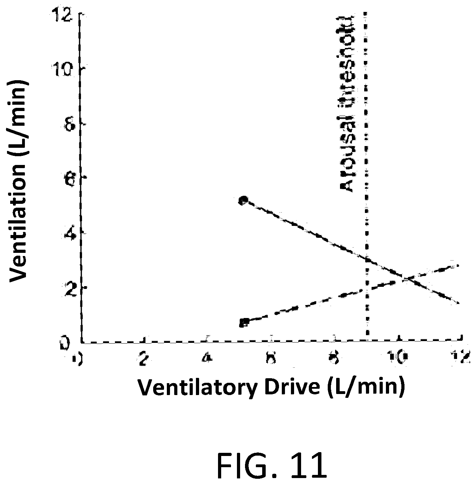

To be able to endotype a patient, data needs to be collected to build a similar figure as show in FIG. 11. FIG. 11 shows the eupneic ventilation, the ventilation during non-obstructed breathing. At this ventilation point the ventilation and ventilatory drive are equal and the breath can be thought of as a normal breath. The black line in the figure has a slope of 1/LG. LG is the loop gain, which is found by dividing the ventilation disturbance by the ventilation response.

In FIG. 11, data used in endotyping a patient with all four physiological traits plotted. The black circle indicates the eupneic ventilation, unobstructed breathing. The black line has a slope equal to 1/LG. The black square marker indicates the ventilation at zero mask pressure. The dashed line has a slope equal to UAG. The dotted line indicates the patient arousal threshold.

In FIG. 11, the black square marker is found by gradually dropping the CPAP pressure to lower levels and measuring the resulting ventilation. A line can be fit to this data and extrapolated to get the ventilation at a CPAP pressure of 0 cmH2O.

The arousal threshold is found by dropping the CPAP to a low-pressure level causing an arousal. The arousal threshold is calculated by fitting a model to the data and estimating the ventilatory drive at arousal.

Loop gain is a parameter in a model of the ventilatory control system which determines how ventilatory drive changes with respect to changes in arterial blood gases. The ventilatory drive is modeled as the sum of the chemical drive, the response to carbon dioxide, and a nonchemical drive accompanying an arousal ${V}_{drive} = {V}_{chem} + {V}_{arousal}$

The chemical drive is described by a differential equation $τ \frac{{dV}_{chem}}{dt} = - {V}_{cham} - {LG}_{0} \times {V}_{E} \left(t-δ\right)$ *τ* is the characteristic time constant due to time course of the buffering of carbon dioxide in the lung and tissue, *LG*₀ is the steady state loop gain, and *V_{E}* is the ventilation. *V_{E}*(*t - δ*) is the previous level of ventilation where the delay *δ* is due to the time delay between the lung and chemoreceptors. *Vₐᵣₒᵤₛₐₗ* is a constant increase in ventilatory drive, *γ*, and accompanies scored EEG arousals.

A method has been introduced to calculate the loop gain, chemical drive, and response to an arousal from flow data and scored arousals. FIG. 12 shows an example of how the introduced algorithm performs. FIG. 12 shows artificial data where the ventilation, purple trace, was created by a model with known parameters. The purple areas indicate periods of an obstruction, and the green line with squares at the top are scored arousals. The black trace is the calculated chemical drive, and the green trace is the ventilatory drive which include the chemical drive plus the response to an arousal. FIG. 12 thus shows a modelled ventilation trace and scored arousals. The purple trace shows the measured ventilation normalized by the ventilation at a normal breath. The shaded areas indicate periods of obstruction, the green line is the calculated ventilatory drive, the black solid curve the calculated chemical drive.

A second method of identifying the loop gain is by identifying the eupneic flow or ventilation, *F_{E}*, during an unobstructed breath. During an airway obstruction the flow or ventilation is reduced. Immediately, the next breath, following an opening in the airway the flow or ventilation will increase dramatically, a recovery breath, *F*_{*n*+}*ᵢ = F_{E} + F_{recovery}*, where *i* is a small number larger than 0 and typically smaller than 3. By calculating the ratio between the response *F_{recovery}*, and the disturbance Δ*F* found by the flow in a breath just preceding the change from an obstructed period to an unobstructed period *F*_{*n*-}*ⱼ = F_{E} -* Δ*F*, where *j* is a small number larger than or equal to 0 and typically smaller than 5. The loop gain can be found by *LG* = *F_{recovery}*/Δ*_{F}.*

Referring to FIG. 10A, a third method of identifying the loop gain is identifying the parameters of the model. During a normal breath the respiratory drive, *Pₘᵤₛ*, and upper airway resistance, *R_{U}*, are identified, and the eupneic ventilation, *F_{E}*, is estimated by calculating the current through the upper airway resistance. During an obstructed breath the drive, *Pₘᵤₛ*, and upper airway resistance, *R_{U}*, are identified and the obstructed ventilation, *F_{obstructed}*, is estimated by calculating the current through the upper airway resistance. The difference between the eupneic ventilation and the obstructed ventilation is the disturbance, *F_{disturbance}* = *F_{E}* - *F_{obstructed}.* By keeping the circuit elements with the values identified during the obstructed breath the value of the upper airway resistance can be changed to the value identified during eupneic breathing. By calculating the current through the upper airway resistance using the *Pₘᵤₛ* identified during the obstructed breathing and the *R_{U}* identified during the eupneic breathing the intended ventilation, *F_{intended}* = *F_{E} + Fᵣₑₛₚₒₙₛₑ*, can be calculated as the sum of the eupneic ventilation and the ventilation response. The loop gain can be found by *LG* = *F_{disturbance}*/*Fᵣₑₛₚₒₙₛₑ.*

The upper airway gain can be identified by comparing the changes in respiratory flow, respiratory drive *Pₘᵤₛ*, and upper airway resistance *Rᵤ*.

One method of identifying the upper airway gain is by identifying periods where breathing is unobstructed. During unobstructed breathing *R_{U}* is the same, or similar, during inhalation and exhalation; or the respiratory drive is linearly proportional to the flow; or the phases of all frequency components of *i_{ab}* are the same as the phases of all frequency components of *iₜₕ.* During the unobstructed periods a baseline drive, *P_{mus E}*, can be identified. At a nearby place in time, where ventilation is obstructed, the flow is reduced to a minimum, *F*₀. Following the reduction in flow the respiratory drive increases and becomes *Pₙ* = *P_{mus E} +* Δ*Pₘᵤₛ.* The increase in respiratory drive causes an increase in flow from the minimum flow *Fₙ = F*₀ *+ ΔF.* The upper airway gain can be found by *UAG =* Δ*F*/Δ*Pₘᵤₛ.*

The arousal threshold can be determined by recording the level of respiratory drive, *Pₘᵤₛ*, just before or at the instance of a recorded arousal.

By comparing the properties of the model to measurements with scored arousals it is possible to construct a metric of the probability of an arousal, Respiratory Arousal Probability (RAP). The RAP could be an indicator of the probability of a certain breath was followed by a cortical arousal, the probability of a certain breath occurring during a cortical arousal, or the probability of a breath following a cortical arousal. The RAP is determined by calculating the elements of the model, or looking at other properties of the RIP signals or flow signals, such as the signal entropy, the ratio of high frequency power to low frequency power, or the relative power in several power bands, and calculating the probability of a breath identified with a set of these parameters to occur before, during, or after an arousal. The method could include combining either the absolute or relative the properties of several consecutive breaths. Therefore, knowing the properties of one or many breaths can predict the probability of a cortical arousal occurring.

A different method of determining the RAP is to measure the time from a breath with certain parameters occurring to the next cortical arousal following the breath. Therefore, knowing the properties of one or many breaths can predict the length of time until next cortical arousal or predict if a cortical arousal will occur within a certain amount of time.

Additional methods for endotyping based on flow and or ventilation values are described in E. Finnsson et al., "A scalable method of determining physiological endotypes of sleep apnea from a polysomnographic sleep study," Sleep, vol. 44, no. 1, p. zsaa168, Jan. 2021, which is incorporated herein in its entirety.

Now, experimental data is presented illustrating the validity of the methods described herein. 71 patients with OSA underwent polysomnographic studies with RIP and oronasal ventilation. To estimate ventilation from RIP, algorithms were applied to handle thoracoabdominal calibration (e.g., the 'powerloss' method), overestimation during paradox, and non-linearity, as discussed above with reference to FIG. 1. Resulting values of RIP ventilation were compared against oronasal ventilation as a gold standard. It was subsequently examined whether RIP provided reliable measures of respiratory event depth and endotypic traits (loop gain, arousal threshold, collapsibility, compensation) via intra-class correlation (ICC) analysis.

The results of this analysis show that Respiratory inductance plethysmography can be used to reliably estimate ventilation and the traits causing OSA. More particularly, RIP calibration and linearization reduced the overestimation of ventilation during paradox (median bias: 7.8 to 0.9 %eupnea) and the underestimation of ventilation during larger breaths (median bias: -20.3 to -6.9 %eupnea). RIP ventilation measures of respiratory event depth (individual event: ICC=0.65 in 15927 events); patient average depth: ICC=0.85) and endotypic traits (ICC range=0.74-0.92) exhibited strong correlations with gold standard measures.

As discussed above, an airflow estimate can be derived from respiratory inductance plethysmography (RIP), which provides a surrogate measure of breathing through thoracic and abdominal movement. Like the nasal cannula, RIP is part of a standard PSG recording and is recommended by the American Academy of Sleep Medicine (AASM) for monitoring respiratory effort and as a secondary airflow sensor. With proper measurement and signal processing, RIP can be used to accurately and reliably measure ventilation independent of breathing route. Importantly, the pneumotach/mask does not interfere with RIP measurements, so simultaneous measurement requires no adjusted setup. Successful validation of endotyping from RIP could therefore make OSA endotyping easily available in the clinical setting. An integral step in this methodology is the calibration of the RIP signal.

The experimental discussed below includes a secondary analysis of a larger observational cohort study to investigate extreme phenotypes of OSA in four participant groups: (1) obese OSA patients, (2) non-obese OSA patients; (3) obese healthy controls, and (4) non-obese healthy controls. Adult male and female participants were recruited from two sites. Patients were excluded from the parent study if they had a sleep disorder diagnosis requiring treatment other than OSA, had periodic limb movements with arousals (>20/hr) or had received surgical or other treatment that affects the upper airway or the gastrointestinal tract. Pregnant patients and those who take sedatives, hypnotics, narcotics, muscle relaxants, or benzodiazepines more than once a week were also excluded.

Patients attended a single in-laboratory overnight polysomnographic sleep study which included 6-channel electroencephalogram, electrocardiogram, pulse oximetry, and thoracic and abdominal respiratory inductance plethysmography (RIP). RIP data are unfiltered (DC-coupled) and linearly related to chest and abdominal circumference. In place of the nasal cannula usually included in clinical polysomnography, quantitative oronasal ventilatory flow was measured by pneumotach via a sealed oronasal mask (Hans Rudolph, Shawnee, Kansas, USA). Sleep stages were manually scored in accordance with AASM guidelines [ref]. Apneas were scored based on a >90% reduction in airflow (oronasal flow). Hypopneas were scored based on a >=30% reduction in airflow (oronasal flow) and >=4% oxygen desaturation. Arousals were scored based on AASM guidelines; arousal start and end times were carefully marked for physiological endotyping (for endotypes: additional arousals that occurred within 3-10 sec of a prior arousal were also scored).

First, an approach to estimating ventilation from RIP was developed and evaluated against oronasal ventilation. Second, the methods to estimate ventilation from RIP was employed to calculate the severity of respiratory events and to estimate the endotypic traits causing OSA (loop gain, arousal threshold, collapsibility, compensation).

Minute ventilation was calculated (tidal volume x respiratory rate) for breaths detected in wake and sleep; values were presented as a percentage of the local average "eupneic" ventilation (7-min window).

For each breath, ventilation was calculated from the RIP signals ('RIP ventilation') without any input from the pneumotach airflow reference signal.

The RIP signals were calibrated using the method described in FIG. 1. That is, three signal processing algorithms were sequentially applied to optimize the estimation of RIP ventilation: First, calibration involves finding the optimal ratio of abdominal to thoracic volume 'k' to sum to provide a surrogate tidal volume signal. Second, overestimation of tidal volumes under circumstances with complete paradox (e.g. during apnea, tidal volume should be minimal but uncorrected RIP can overestimate ventilation) was corrected for. Third, non-linearity in the form of underestimation of tidal volumes during larger breaths was corrected for.

RIP ventilation values were normalized for further analysis as a percentage of the local average "eupneic" RIP ventilation (7-min window).

The depth of each respiratory event (hypopneas and apneas; scored using oronasal ventilation) was calculated using RIP ventilation for comparison against event depth calculated separately from oronasal ventilation. Event depth was calculated for each manually scored respiratory event as the "nadir ventilation"; e.g. an event depth of 30% represents a 30% reduction in flow (from the moving-time mean 'eupneic' baseline level) and is the minimum flow-reduction requirement for a scored respiratory event (hypopnea). Adequate characterization of event depth was used for defining the clinically-scored events that define OSA. In addition, the average respiratory event depth for each subject was calculated; greater average event depth is a measure of greater upper airway collapsibility and a phenotypic biomarker of more severe OSA.

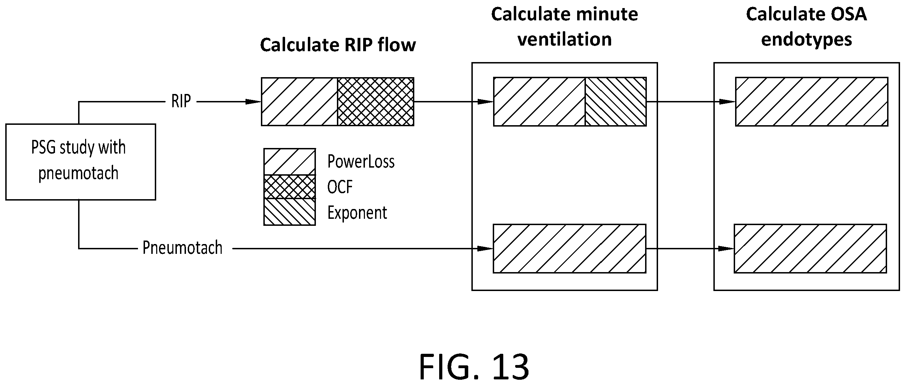

FIG. 13 illustrates a process for comparing/validating the RIP derived flow and endotypes against those derived via pneumotach. The OSA endotypes were calculated using RIP ventilation for comparison with separately-calculated traits using oronasal ventilation. OSA endotypes were calculated as described in E. Finnsson et al., "A scalable method of determining physiological endotypes of sleep apnea from a polysomnographic sleep study," Sleep, vol. 44, no. 1, p. zsaa168, Jan. 2021. Briefly, a physiological model was fit to the ventilation signal (for each 7-min window) between scored respiratory events to describe the ventilatory control system (gain, response time, delay), calculate the "loop gain" (value presented at 1 cycle/min), and provide an estimate of ventilatory drive. "Arousal threshold" was then calculated as the value of ventilatory drive preceding scored arousals. Median values of loop gain and the arousal threshold during eligible windows (at least 1 respiratory event, contains non-REM sleep, contains no REM sleep, contains no more than 30 s periods of wake) provided representative values for each patient. Collapsibility and compensation were calculated by first sorting all values of ventilatory drive during sleep (excluding breaths within arousals/wake, non-REM only) into multiple quantiles (N=20). Each quantile was characterized by median ventilatory drive and median ventilation values to provide a 20-point plot of ventilation vs. ventilatory drive, so called endogram. "Vpassive" was calculated as the ventilation at normal ventilatory drive (100 %eupnea) and taken to represent collapsibility (lower values of ventilation reflect greater collapsibility). "Compensation" was calculated as the increase in ventilation (from Vpassive) when ventilatory drive rises to maximal levels during sleep (i.e. to the arousal threshold).

In the statistical analysis, regarding the RIP paradox overestimation, the "RIP paradox" was defined as breaths with -1.0 < R < -0.8. The median bias for breaths in this is reduced after applying OCF. Regarding the RIP non-linearity, large breaths were defined as breaths where minute ventilation was > 100%eupnea. Median large breath bias should be reduced after applying power law scaling. Regarding the event depth, the agreement between the event depth derived from 1) the calibrated RIP signals and 2) the oronasal pneumotachograph was evaluated using intraclass correlation (ICC). For individual breaths as well as per patient mean event depth. Regarding the normalization, arousal threshold and Vpassive values were projected onto a normal distribution using a square-root transform ArThres=1+(ArThres - 1)0.5 and Vpassive=1-(1-Vpassive)0.5. Regarding the OSA endotypes, the agreement between the endotypes derived from 1) the calibrated RIP signals and 2) the oronasal pneumotachograph was investigated by using ICC, and Bland-Altman (BA) mean error and agreement. Intraclass correlation coefficient (ICC) was calculated using two-way mixed effects, with a single rater and absolute agreement. Confidence intervals were calculated using bootstrap, randomly sampling sleep studies with replacement for 10.000 iterations. The following table (Table 1) illustrates participant characteristics for the experimental study.

**Table 1. Cohort information**

| | | Participants with OSA |
|---|---|---|
| N | | 71 |
| Gender (M/F) | | 58/13 |
| Ethnicity | | |
| | Caucasian | 57 |
| | African-American | 10 |
| | Asian | 3 |
| | Other | 1 |
| AHI (mean/SD) [events/hour] | | 37.38 (28.08) |
| Age (mean/SD) [yrs] | | 53.35 (11.33) |
| BMI (mean/SD) [kg/m2] | | 34.19 (6.66) |
| mean REM % duration (stdv) | | 10.63 (8.01) |
| mean N1 % duration (stdv) | | 27.56 (18.76) |
| mean N2 % duration (stdv) | | 50 (13.15) |
| mean N3 % duration (stdv) | | 11.82 (10.00) |
| mean time in supine in minutes (stdv) | | 55.53 (32.32) |
| mean TST in minutes (stdv) | | 337.93 (99.29) |
| fraction in hypopnea HI/AHI (stdv) | | 0.58 (0.31) |
| fraction in central apnea (stdv) | 0.03 (0.06) | |
| fraction of events ending in arousal* | 0.39 (0.08) | |

| | | |
|---|---|---|
| *An event was considered to end in arousal if an arousal occurred within 10 seconds of the end of the event | | |

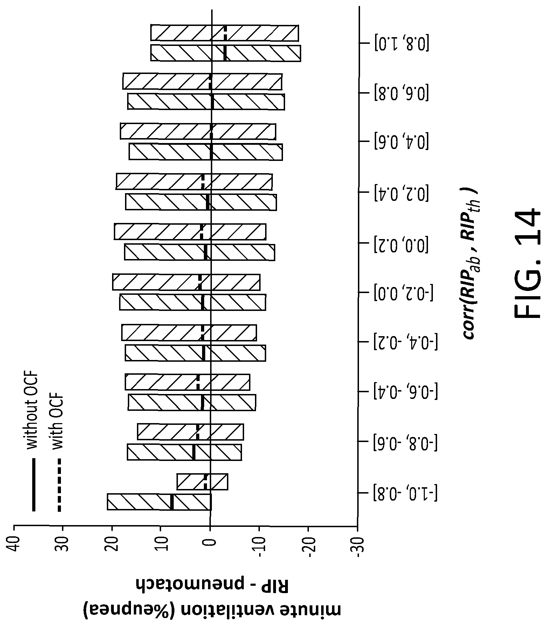

FIG. 14 illustrates experimental results for the RIP paradox overestimation for results with and without applying the OCF. More particularly, FIG. 14 shows the absolute minute ventilation error before and after applying OCF. As can be seen, the OCF reduces the median bias of RIP ventilation during periods of paradoxical breathing from 7.8%eupnea to 0.9%eupnea (Figure 4). A total of 381.812 breaths were analyzed, 20.707 of which were in the (-1, -0.8) bin.

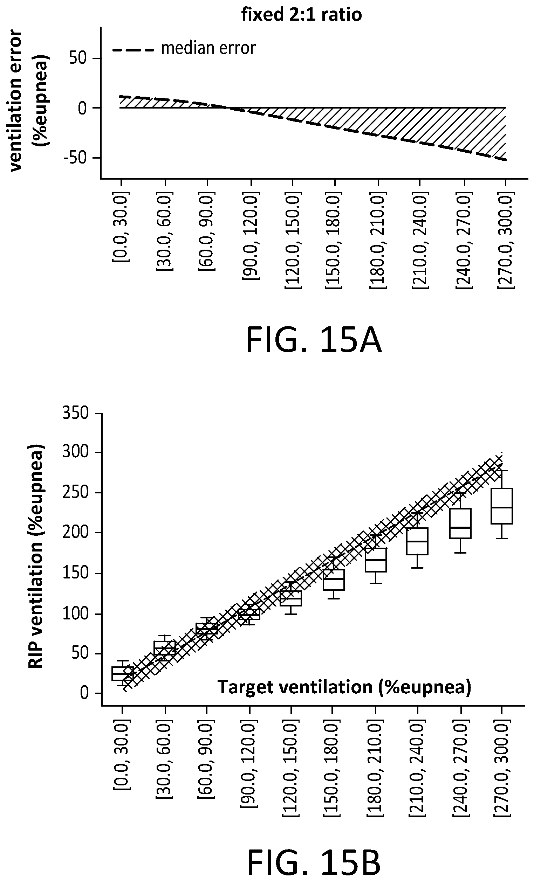

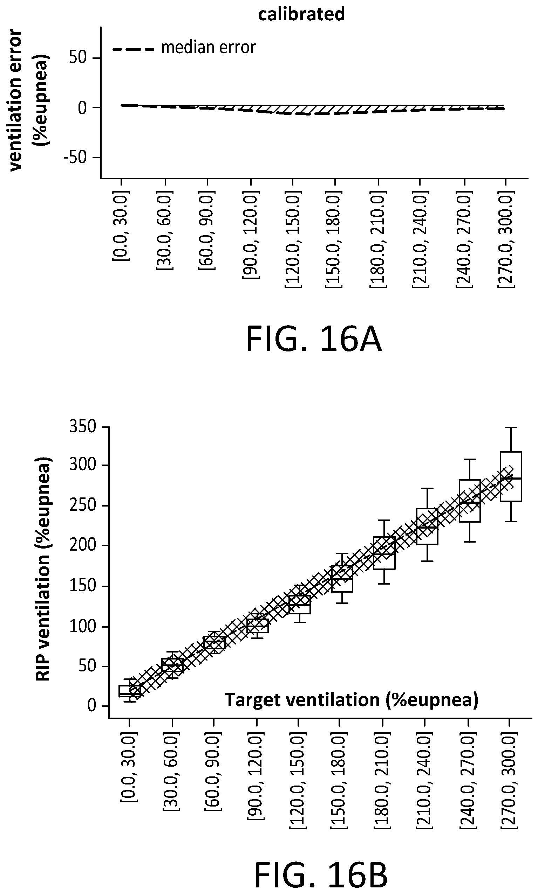

FIGs.15A,15B, 16A, and 16B illustrate experimental results related to the RIP non-linearity. FIGs.15A and15B show the results without the non-linearity correction, and FIGs.16A and16B show the results with the non-linearity correction. More particularly, these figures show the effects of calibration on different ranges of ventilation from small breaths to the left to large breaths to the right. In FIGs. 15B and 16B, the dashed line is the line of unity y=x and the shaded region shows the bin edges. In FIGs. 15A and 16A, the grey line shows the median error within each drive bin and the shaded region represents the median absolute error within each drive bin. As can be seen, the median large breath bias is reduced from: - 20.3 to -6.9 %eupnea. A large breath is defined as all breaths where ventilation > 100%eupnea.

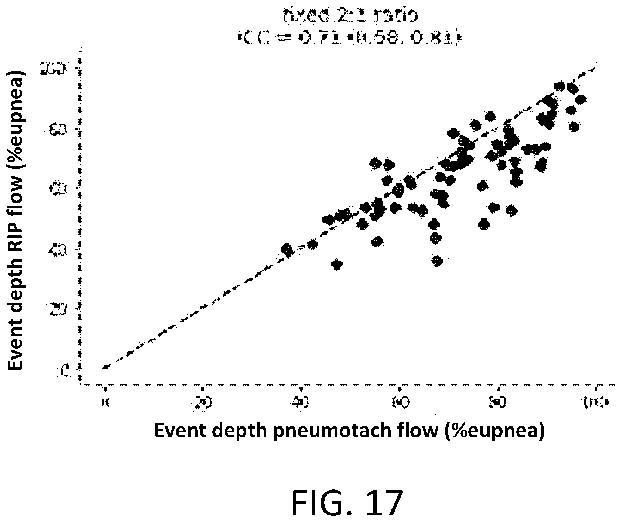

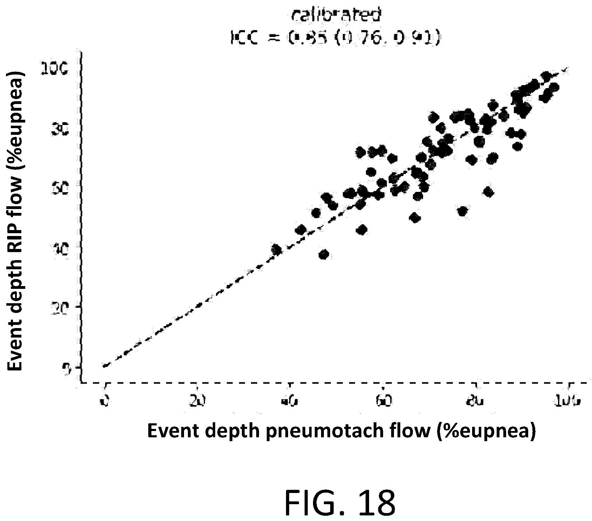

FIGs. 17 and 18 illustrate experimental results related to the event depth. FIG. 17 illustrates results for a fixed value of the calibration constant k, and FIG. 18 illustrates results when the calibration constant k is determined using the above-described methods. More particularly, these figures show the average event depth for n=71 sleep studies. The dashed line on the scatter plots is the line of identity (y=x). Each figure also contains the ICC of between the two methods of deriving average event depths with the 95% confidence interval. In FIG. 17, the calibration constant k, which is a fixed 2:1 ratio, resulted in an ICC value of 0.71 (0.58, 0.81), median (95p CI). In FIG. 18, the calibrated calibration constant k resulted in an ICC value of 0.85 (0.76, 0.91).

Table 2 summarize result for the OSA endotypes. The calibrated RIP derived OSA endotype values are compared to the values derived from the gold standard oronasal pneumotach. Table 2 shows the summary statistics for each endotype. For all endotypes, there is high agreement between the two derivation methods.

**Table 2. Summary statistics describing the agreement between RIP endotypes after POE calibration of the RIP signals and pneumotach endotypes: Intraclass Correlation Coefficient (ICC), Pearson correlation coefficient (PCC), Bland-Altman bias and limits of agreement (Pneumotach - RIP) and mean absolute error as well as the mean absolute error.**

| | ICC | PCC | Mean error | 95% agr. interv. | mean abs error |
|---|---|---|---|---|---|
| ArThres | 0.92 (0.87,0.95) | 0.92 (..., ...) | -1.29 (..., ...) | 23.24 (..., ...) | 9.40 (..., ...) |
| V active | 0.88 (..., ...) | 0.89 (..., ...) | -5.40 (..., ...) | 32.30 (..., ...) | 12.25 (..., ...) |
| Vpassive | 0.92 (..., ...) | 0.92 (..., ...) | 1.45 (..., ...) | 18.61 (..., ...) | 7.04 (..., ...) |
| Vcomp | 0.74 (..., ...) | 0.76 (..., ...) | -5.20 (..., ...) | 31.03 (..., ...) | 11.95 (..., ...) |
| LG1 | 0.78 (..., ...) | 0.89 (..., ...) | 0.06 (..., ...) | 0.27 (..., ...) | 0.12 (..., ...) |

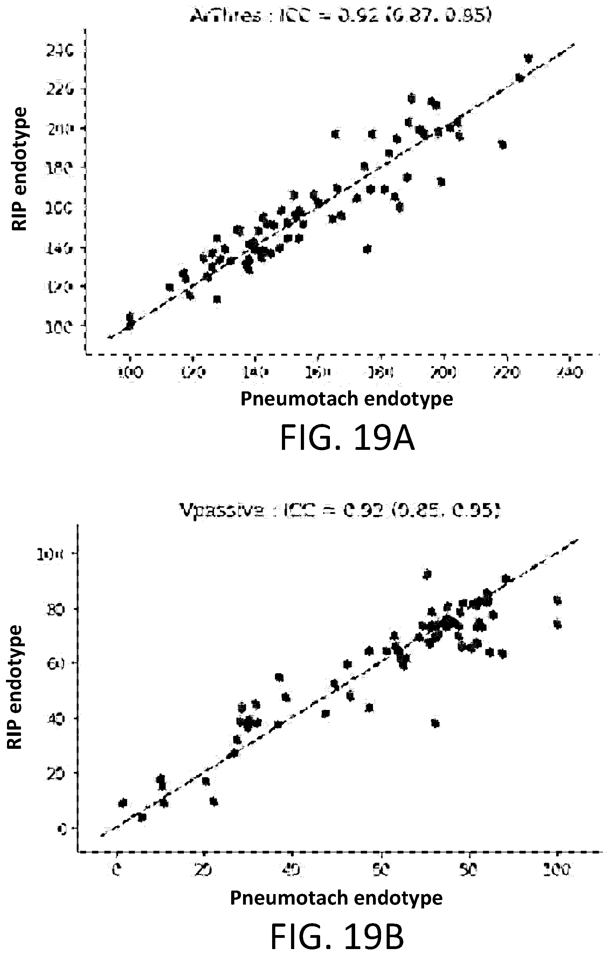

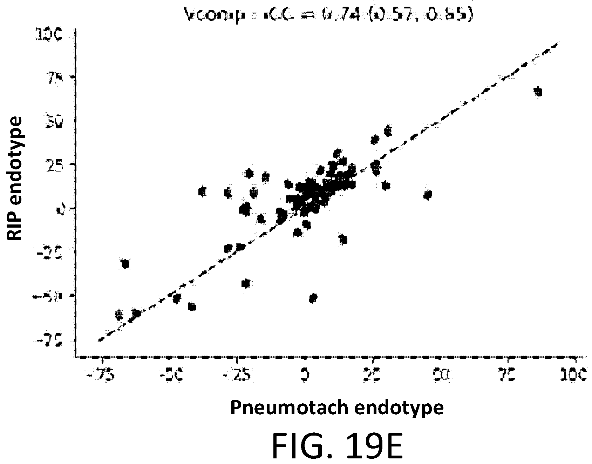

FIGs. 19A-19E 5 show the ICC values for the comparison of the endotypes derived using RIP measurements versus endotypes derived using pneumotach measurements. The ICC values for the endotypes were found to be 0.92 for arousal threshold, 0.78 for LG1, 0.92 for Vpassive, 0.88 for Vactive, and 0.74 for Vcomp. More particularly, these figures compare the endotypes as derived from the two methods of measurement RIP vs. the gold standard oronasal pneumotach. Each datapoint represents a full sleep study of a single participant. The dashed line on the scatter plots is the line of identity (y=x). Each figure also contains the ICC of the respective endotype with the corresponding 95% confidence interval.

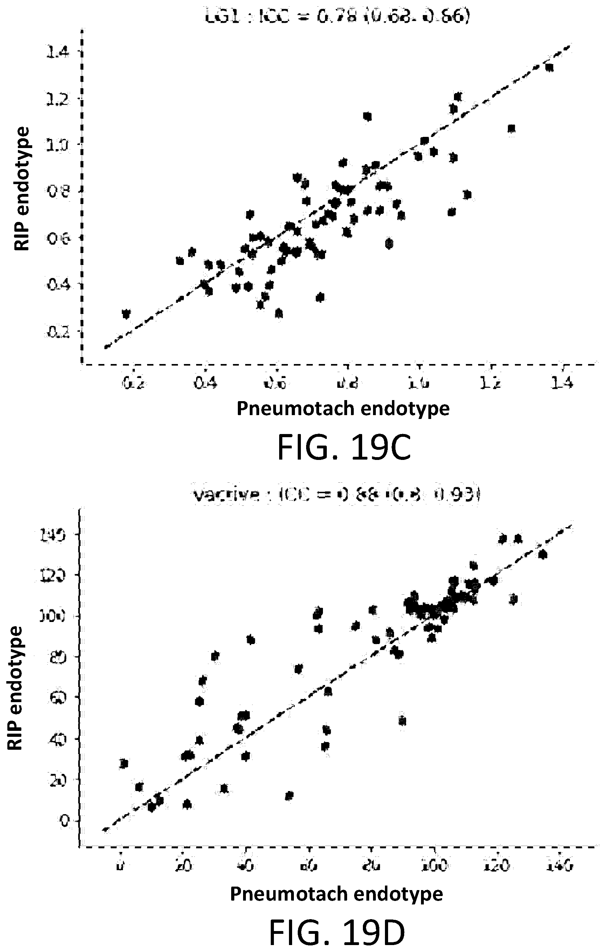

FIG. 19A shows the comparison for the arousal threshold endotype. FIG. 19B shows the comparison for the upper airway collapsibility endotype. FIG. 19C shows the comparison for the loop gain endotype. FIG. 19D shows the comparison for the ventilation at the arousal threshold endotype. FIG. 19D shows the comparison for the upper airway muscle compensation endotype.

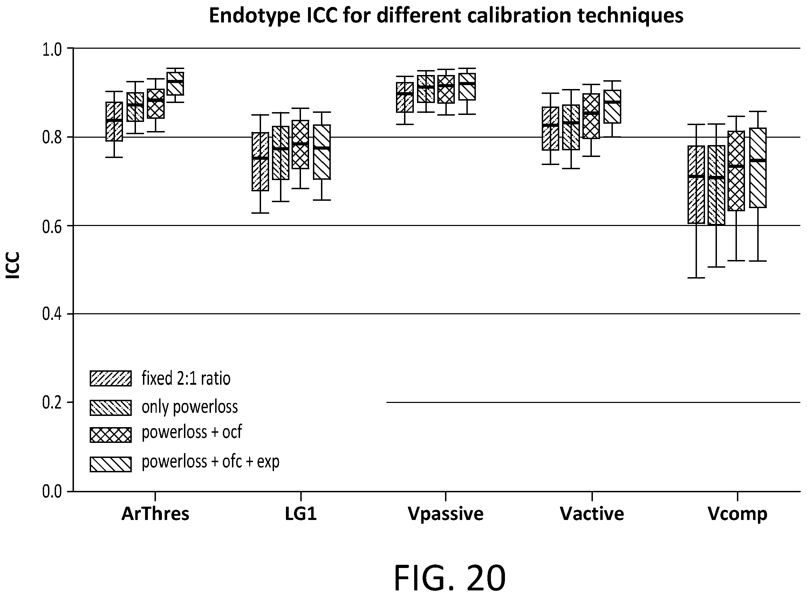

FIG. 20 shows ICC between RIP derived endotypes and the gold standard pneumotach derived endotypes for different subsets of the RIP calibration technique. Confidence intervals were calculated using bootstrapping. The ICC was calculated for each endotype using different levels of calibration on the RIP signals. Figure 6 shows boxplots of the ICC values when the RIP is calibrated using the 1:2 ratio, PowerLoss, PowerLoss and OCF, and PowerLoss, OCF and exponential scaling. The figure shows how the calibration methods impact the ICC.

As can be seen in the experimental results, OSA endotypes can be successfully derived from PSG sleep studies using RIP belts to measure minute ventilation. Further, using the methods described herein, the limitations of linear RIP calibration methods can be supplemented using nonlinear calibration methods.

The methods described herein improve the accessibility of OSA endotyping by developing a RIP calibration method that allows for the extraction of endotypes from the RIP signals. The above experimental results demonstrate this, as shown by the high agreement between RIP and pneumotach derived endotypes, for all explored OSA endotypes (upper airway collapsibility, upper airway muscle compensation, arousal threshold and loop gain).

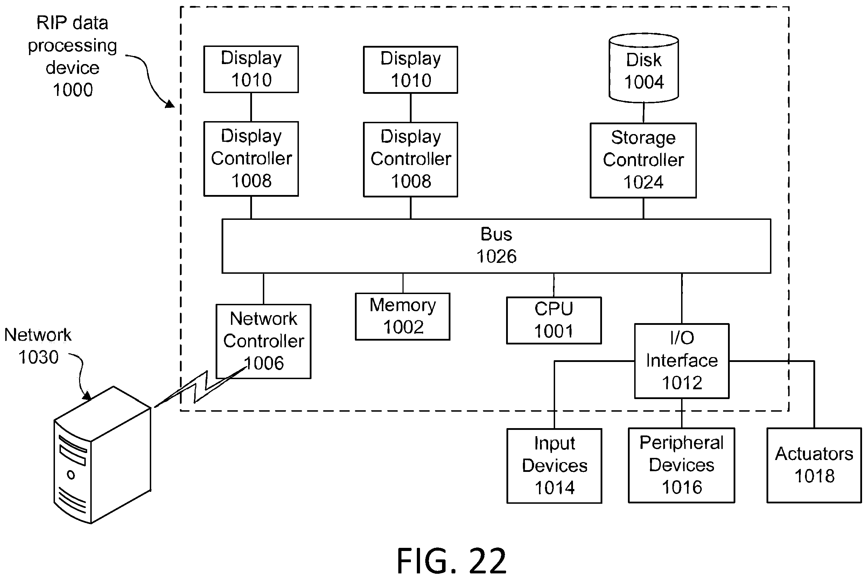

FIG. 22 illustrates a device 1000 configured to perform method 100 and process the RIP data to generate flow values, ventilation values, and/or endotypes. The device 1000 can perform some or all of the steps discussed above. The device 1000 may perform method 100 using a utility application, background daemon, or component of an operating system, or combination thereof, executing in conjunction with CPU 1001 and an operating system such as Microsoft Windows 7, UNIX, Solaris, LINUX, Apple MAC-OS and other systems known to those skilled in the art.

CPU 1001 may be a Xenon or Core processor from Intel of America or an Opteron processor from AMD of America, or may be other processor types that would be recognized by one of ordinary skill in the art. Alternatively, the CPU 1001 may be implemented on an FPGA, ASIC, PLD or using discrete logic circuits, as one of ordinary skill in the art would recognize. Further, CPU 1001 may be implemented as multiple processors cooperatively working in parallel to perform the instructions of the inventive processes described above.

The device 1000 in FIG. 22 also includes a network controller 1006, such as an Intel Ethernet PRO network interface card from Intel Corporation of America, for interfacing with a network 1030. The network 1030 can be a public network, such as the Internet, or a private network such as an LAN or WAN network, or any combination thereof and can also include PSTN or ISDN sub-networks. The network 1030 can also be wired, such as an Ethernet network, or can be wireless such as a cellular network including EDGE, 3G and 4G wireless cellular systems. The network 1030 can also be Wi-Fi, Bluetooth, or any other wireless form of a communication that is known.

The device 1000 further includes a display controller 1008 for interfacing with a display 1010. A general purpose I/O interface 1012 interfaces with input devices 1014 as well as peripheral devices 1016. The general purpose I/O interface also can connect to a variety of actuators 1018. The input devices 1014 can include the various sensors shown in FIGs. 3 and 4, for example. The input devices 1014 may include an interface to receive data from the signal processor 350 in FIG. 3, for example.

A sound controller 1020 may also be provided in the device 1000 to interface with speakers/microphone 1022 thereby providing sounds and/or music.

A general purpose storage controller 1024 connects the storage medium disk 1004 with a communication bus 1026, which may be an ISA, EISA, VESA, PCI, or similar, for interconnecting all of the components of the device 1000. Descriptions of general features and functionality of the display 1010, input devices 1014 (e.g., a keyboard and/or mouse), as well as the display controller 1008, storage controller 1024, network controller 1006, sound controller 1020, and general purpose I/O interface 1012 are omitted herein for brevity as these features are known.

Method 100 can be stored on computer storage media and performed using a computation/logic circuitry. For example, method 100 may be performed on a central processing unit (CPU). Computer storage media are physical storage media that store computer-executable instructions and/or data structures. Physical storage media include computer hardware, such as RAM, ROM, EEPROM, solid state drives ("SSDs"), flash memory, phase-change memory ("PCM"), optical disk storage, magnetic disk storage or other magnetic storage devices, or any other hardware storage device(s) which can be used to store program code in the form of computer-executable instructions or data structures, which can be accessed and executed by a general-purpose or special-purpose computer system to implement the disclosed functionality of the disclosure.

Transmission media can include a network and/or data links which can be used to carry program code in the form of computer-executable instructions or data structures, and which can be accessed by a general-purpose or special-purpose computer system. A "network" may be defined as one or more data links that enable the transport of electronic data between computer systems and/or modules and/or other electronic devices. When information is transferred or provided over a network or another communications connection (either hardwired, wireless, or a combination of hardwired or wireless) to a computer system, the computer system may view the connection as transmission media. Combinations of the above should also be included within the scope of computer-readable media.

Further, upon reaching various computer system components, program code in the form of computer-executable instructions or data structures can be transferred automatically from transmission media to computer storage media (or vice versa). For example, computer-executable instructions or data structures received over a network or data link can be buffered in RAM within a network interface module (e.g., a "NIC"), and then eventually transferred to computer system RAM and/or to less volatile computer storage media at a computer system. Thus, it should be understood that computer storage media can be included in computer system components that also (or even primarily) utilize transmission media.

Computer-executable instructions may comprise, for example, instructions and data which, when executed by one or more processors, cause a general-purpose computer system, special-purpose computer system, or special-purpose processing device to perform a certain function or group of functions. Computer-executable instructions may be, for example, binaries, intermediate format instructions such as assembly language, or even source code.

The disclosure of the present application may be practiced in network computing environments with many types of computer system configurations, including, but not limited to, personal computers, desktop computers, laptop computers, message processors, hand-held devices, multi-processor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. As such, in a distributed system environment, a computer system may include a plurality of constituent computer systems. In a distributed system environment, program modules may be located in both local and remote memory storage devices.

The disclosure of the present application may also be practiced in a cloud-computing environment. Cloud computing environments may be distributed, although this is not required. When distributed, cloud computing environments may be distributed internationally within an organization and/or have components possessed across multiple organizations. In this description and the following claims, "cloud computing" is defined as a model for enabling on-demand network access to a shared pool of configurable computing resources (e.g., networks, servers, storage, applications, and services). The definition of "cloud computing" is not limited to any of the other numerous advantages that can be obtained from such a model when properly deployed.

A cloud-computing model can be composed of various characteristics, such as on-demand self-service, broad network access, resource pooling, rapid elasticity, measured service, and so forth. A cloud-computing model may also come in the form of various service models such as, for example, Software as a Service ("SaaS"), Platform as a Service ("PaaS"), and Infrastructure as a Service ("IaaS"). The cloud-computing model may also be deployed using different deployment models such as private cloud, community cloud, public cloud, hybrid cloud, and so forth.

Some embodiments, such as a cloud-computing environment, may comprise a system that includes one or more hosts that are each capable of running one or more virtual machines. During operation, virtual machines emulate an operational computing system, supporting an operating system and perhaps one or more other applications as well. In some embodiments, each host includes a hypervisor that emulates virtual resources for the virtual machines using physical resources that are abstracted from view of the virtual machines. The hypervisor also provides proper isolation between the virtual machines. Thus, from the perspective of any given virtual machine, the hypervisor provides the illusion that the virtual machine is interfacing with a physical resource, even though the virtual machine only interfaces with the appearance (e.g., a virtual resource) of a physical resource. Examples of physical resources including processing capacity, memory, disk space, network bandwidth, media drives, and so forth.

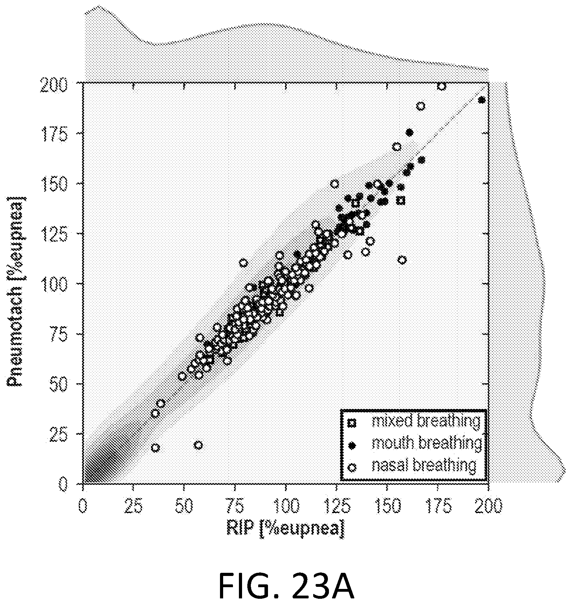

FIG. 23A illustrates a comparison in the determined event depths for three sets of data corresponding to mouth breathing, nasal breathing, and mixed breathing. The vertical axis represents the event depth determined based on pneumotach measurements. The horizontal axis represents the event depth determined based on RIP measurements. Close agreement between the approaches to determining the event depth is shown by the clustering of these results near the x=y line. Because pneumotach is taken as the gold standard, this close agreement indicates that RIP measurements provide an accurate approach to determine event depth.

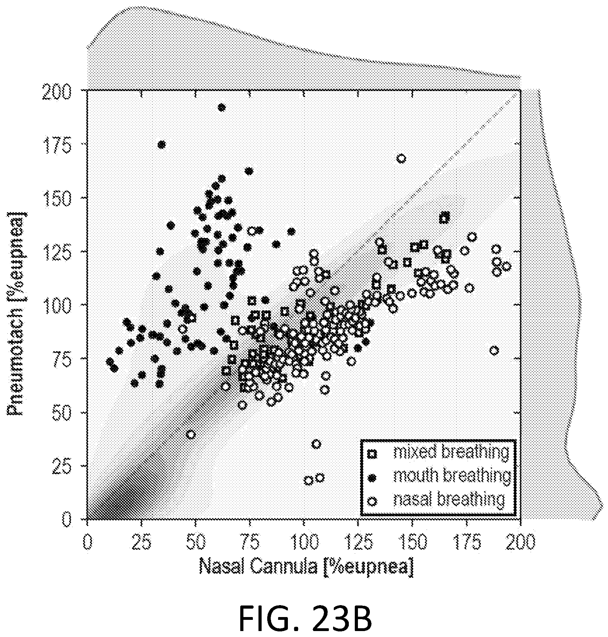

FIG. 23B illustrates a comparison in the determined event depths for three sets of data corresponding to mouth breathing, nasal breathing, and mixed breathing. The vertical axis represents the event depth determined based on pneumotach measurements. The horizontal axis represents the event depth determined based on nasal cannula measurements. As might be expected, the nasal-cannula-based ventilation underestimate the ventilation for the mouth breathing data points. Thus, the RIP-based ventilation results appear to be better for predicting ventilation.

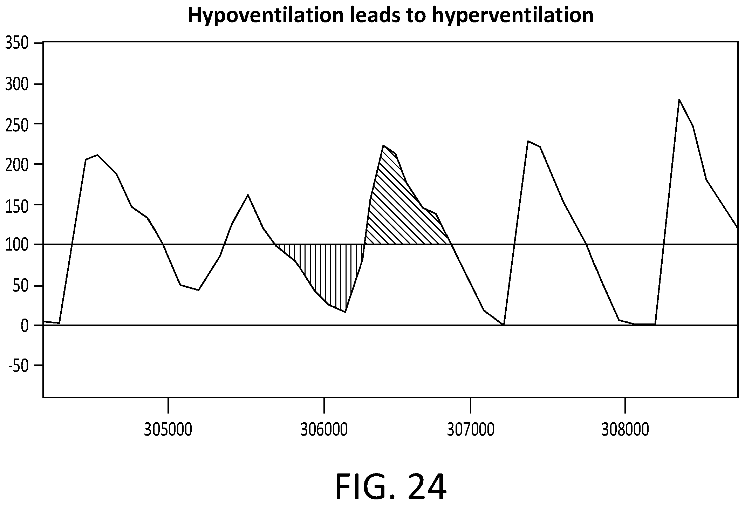

FIG. 24 illustrates ventilation with respect to time. A cycle of hypoventilation (indicated by the vertical stripes) followed by hyperventilation (indicated by the diagonal stripes) is shown. Hyperventilation allows recovery from the oxygen deprivation occurring during hypoventilation.

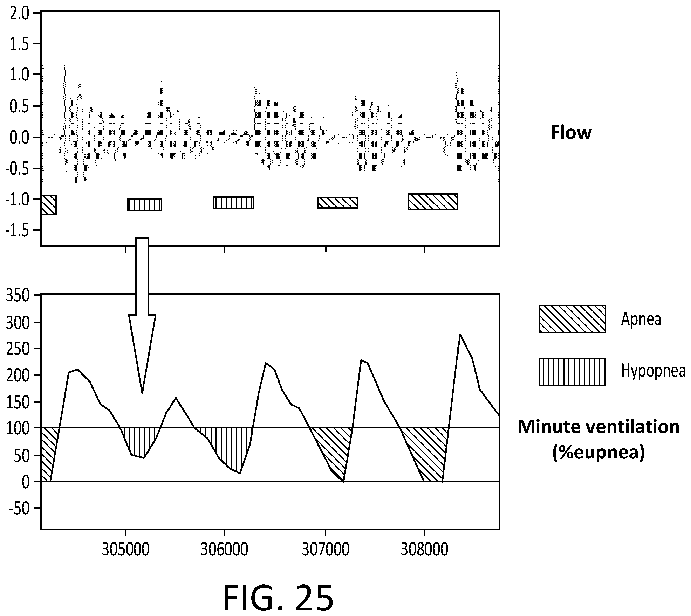

FIG. 25 illustrates flow and ventilation with respect to time. More particularly, FIG. 25 illustrates periods of apnea and hypopnea. Apnea is an OSA in which there occurs a full obstruction of the airway when a person is asleep. Hypopnea is an OSA in which there occurs a partial blockage of the airway.

Certain terms are used throughout the description and claims to refer to particular methods, features, or components. As those having ordinary skill in the art will appreciate, different persons may refer to the same methods, features, or components by different names. This disclosure does not intend to distinguish between methods, features, or components that differ in name but not function. The figures are not necessarily drawn to scale. Certain features and components herein may be shown in exaggerated scale or in somewhat schematic form and some details of conventional elements may not be shown or described in interest of clarity and conciseness.

Although various example embodiments have been described in detail herein, those skilled in the art will readily appreciate in view of the present disclosure that many modifications are possible in the example embodiments without materially departing from the concepts of present disclosure. Accordingly, any such modifications are intended to be included in the scope of this disclosure. Likewise, while the disclosure herein contains many specifics, these specifics should not be construed as limiting the scope of the disclosure or of any of the appended claims, but merely as providing information pertinent to one or more specific embodiments that may fall within the scope of the disclosure and the appended claims. Any described features from the various embodiments disclosed may be employed in combination. In addition, other embodiments of the present disclosure may also be devised which lie within the scopes of the disclosure and the appended claims. Each addition, deletion, and modification to the embodiments that falls within the meaning and scope of the claims is to be embraced by the claims.

Certain embodiments and features may have been described using a set of numerical upper limits and a set of numerical lower limits. It should be appreciated that ranges including the combination of any two values, e.g., the combination of any lower value with any upper value, the combination of any two lower values, and/or the combination of any two upper values are contemplated unless otherwise indicated. Certain lower limits, upper limits and ranges may appear in one or more claims below. Any numerical value is "about" or "approximately" the indicated value, and takes into account experimental error and variations that would be expected by a person having ordinary skill in the art.

This disclosure provides various examples, embodiments, and features which, unless expressly stated or which would be mutually exclusive, should be understood to be combinable with other examples, embodiments, or features described herein.

## Claims

1. A computer-implemented method for determining a respiratory flow from data from respiratory inductance plethysmography (RIP) signals, the method comprising:
receiving data of a thoracic signal of a first RIP belt arranged proximate with a thorax of a subject;
receiving data of an abdomen signal of a second RIP belt arranged proximate with an abdomen of the subject; and
determining a respiratory flow of the subject based on the data of the thoracic signal and the data of the abdomen signal,
wherein determining the respiratory flow includes two or more calibrations, including:
performing a first calibration by applying a first calibration coefficient that relates an amplitude of a differential change in the thoracic signal to an amplitude of a differential change in the abdomen signal to obtain a determined respiratory flow, and
performing a second calibration on the determined respiratory flow that corrects for a non-linearity in the determined respiratory flow.

2. The method according to claim 1, further comprising a step of determining one or more endotypes of an obstructive sleep apnea or of another sleep disorder of the subject based on the determined, calibrated respiratory flow.

3. The method according to any one of the claims 1 - 2, wherein determining the respiratory flow further includes a third calibration that corrects for an overestimation of the respiratory flow during a paradox.

4. The method according to any one of the claims 1 - 3, wherein the method further comprises taking respective derivatives of the thoracic signal and the abdomen signal and combining the derivatives using a scaling coefficient to determine the respiratory flow.

5. The method according to any one of the claims 1 - 4**,** wherein determining of the respiratory flow includes
calculating a change of the thoracic signal with respect to time to generate a derivative corresponding to a time derivative of a thoracic volume,
calculating a change of the abdomen signal with respect to time to generate another derivative corresponding to a time derivative of an abdomen volume, and
the respiratory flow is determined by combining the derivative and the another derivative using a weighted sum that is based on the first calibration coefficient.

6. The method according to any one of the claims 1 - 5, wherein the determining of the respiratory flow includes calculating a time derivative of a calibrated sum of the thoracic signal and the abdomen signal, the calibrated sum being based on the first calibration coefficient.

7. The method according to any one of the claims 1 - 6, wherein the first calibration is determined using the thoracic signal and the abdomen signal in an absence of all respiratory flow signals, and/or
wherein the first calibration is determined using the thoracic signal and the abdomen signal in an absence of respiratory flow signals measured by one or more nasal canula, and/or
wherein the first calibration is carried out using a PowerLoss calibration method.

8. The method according to any one of the claims 1 - 7, wherein the first calibration is carried out by selecting a value of the calibration coefficient that minimizes a function that represents a ratio of numerator to a denominator,
the numerator being a power of a weighted/scaled sum of the thoracic signal and the abdomen signal, wherein a weighting/scaling of the weighted/scaled sum is based on the value of the calibration coefficient,
the denominator being a power of the scaled thoracic signal summed with a power of a weighted/scaled abdomen signal, which is the abdomen signal that has been weighted/scaled sum is based on the value of the calibration coefficient,
wherein a weighting/scaling factor used to weight/scale the thoracic signal relative to the abdomen signal is the calibration coefficient or a function of the calibration coefficient.

9. The method according to any one of the claims 1 - 8, wherein the first calibration is carried out by finding the value of k that satisfies to a predetermined threshold the equation ${min}_{k} \frac{RMS \left(\left(1-k\right){dRIP}_{th}+k {dRIP}_{ab}\right)}{RMS \left(\left(1-k\right){dRIP}_{th}\right) + RMS \left(k {dRIP}_{ab}\right)} ,$ wherein RMS is the root mean square, k is a test value of the calibration coefficient, dRIPₜₕ represents a derivative of the thoracic signal with respect to time, dRIP_{ab} represents a derivative of the abdomen signal with respect to time.

10. The method according to claim 3, wherein the third calibration includes steps of
determining a correlation factor between the thoracic signal and the abdomen signal, and
calculating an overestimation correction factor based on the correlation factor, and
wherein the determining of the respiratory flow further includes scaling a derivative of a weighted sum of the thoracic signal and the abdomen signal by the overestimation correction factor, and/or wherein the determining of the respiratory flow further includes scaling the thoracic signal and the abdomen signal by the overestimation correction factor.

11. The method according to any one of the claims 1 - 10, wherein the second calibration includes steps of
calculating a non-linearity correction factor based on a curve fit, the curve fit having been generated from calibration data that includes the respiratory flow, which is uncorrected for the non-linearity, and a reference flow, which is measured using trusted/validated method and is acquired concurrently with the respiratory flow of the calibration data, and
wherein the determining of the respiratory flow further includes scaling a derivative of a weighted sum of the thoracic signal and the abdomen signal by the non-linearity correction factor, and/or wherein the determining of the respiratory flow further includes scaling the thoracic signal and the abdomen signal by the non-linearity correction factor.

12. The method according to claim 11, wherein the curve fit for the calculating of the non-linearity correction factor has an exponential functional form and the non-linearity correction factor is an exponent.

13. A computer readable medium having stored thereon instructions that, when executed by one or more processors of a computing system cause the one or more processors to execute the steps of the method according to any one of the claims 1 - 12.

14. A device for determining a respiratory flow from data from respiratory inductance plethysmography (RIP) signals, the device comprising:
a processor configured to
receive data of a thoracic signal of a first RIP belt arranged proximate with a thorax of a subject;
receive data of an abdomen signal of a second RIP belt arranged proximate with an abdomen of the subject; and
determine a respiratory flow of the subject based on the data of the thoracic signal and the data of the abdomen signal,
wherein determining the respiratory flow includes two or more calibrations, includes:
performing a first calibration by applying a first calibration coefficient that relates an amplitude of a differential change in the thoracic signal to an amplitude of a differential change in the abdomen signal to obtain a determined respiratory flow, and
performing a second calibration on the determined respiratory flow that corrects for a non-linearity in the determined respiratory flow.

15. A system comprising:
a plurality of respiratory inductance plethysmography (RIP) belts, including a thoracic belt configured to obtain a thoracic signal and an abdomen belt configured to obtain an abdomen signal; and
a processor configured to
receive data of the thoracic signal of a first RIP belt arranged proximate with a thorax of a subject;
receive data of the abdomen signal of a second RIP belt arranged proximate with an abdomen of the subject; and
determine a respiratory flow of the subject based on the data of the thoracic signal and the data of the abdomen signal,
wherein determining the respiratory flow includes two or more calibrations, includes:
performing a first calibration by applying a first calibration coefficient that relates an amplitude of a differential change in the thoracic signal to an amplitude of a differential change in the abdomen signal to obtain a determined respiratory flow, and
performing a second calibration on the determined respiratory flow that corrects for a non-linearity in the determined respiratory flow.

## Patentansprüche

1. Ein computerimplementiertes Verfahren zur Bestimmung eines respiratorischen Flusses aus Daten von Signalen der Respiratorischen Induktionsplethysmographie (RIP), wobei das Verfahren umfasst:
Empfangen von Daten eines thorakalen Signals eines ersten RIP-Gurts, der in Nähe des Thorax eines Subjekts angeordnet ist;
Empfangen von Daten eines Abdomensignals eines zweiten RIP-Gurts, der in Nähe des Abdomens des Subjekts angeordnet ist; und
Bestimmen eines respiratorischen Flusses des Subjekts auf der Grundlage der Daten des thorakalen Signals und der Daten des Abdomensignals,
wobei das Bestimmen des respiratorischen Flusses zwei oder mehr Kalibrierungen umfasst, einschließlich:
Durchführen einer ersten Kalibrierung durch Anwenden eines ersten Kalibrierungskoeffizienten, der eine Amplitude einer differentiellen Änderung im thorakalen Signal mit einer Amplitude einer differentiellen Änderung im Abdomensignal in Beziehung setzt, um einen bestimmten respiratorischen Fluss zu erhalten, und
Durchführen einer zweiten Kalibrierung an dem bestimmten respiratorischen Fluss, die eine Nichtlinearität in dem bestimmten respiratorischen Fluss korrigiert.

2. Verfahren, nach Anspruch 1, ferner umfassend einen Schritt des Bestimmens eines oder mehrerer Endotypen einer obstruktiven Schlafapnoe oder einer anderen Schlafstörung des Subjekts auf der Grundlage des bestimmten, kalibrierten respiratorischen Flusses.

3. Verfahren, nach einem der Ansprüche 1 - 2, wobei das Bestimmen des respiratorischen Flusses ferner eine dritte Kalibrierung umfasst, die eine Überschätzung des respiratorischen Flusses während eines Paradox korrigiert.

4. Verfahren, nach einem der Ansprüche 1 - 3, wobei das Verfahren ferner umfasst, jeweilige Ableitungen des thorakalen Signals und des Abdomensignals zu bilden und die Ableitungen unter Verwendung eines Skalierungskoeffizienten zu kombinieren, um den respiratorischen Fluss zu bestimmen.

5. Verfahren, nach einem der Ansprüche 1 - 4, wobei das Bestimmen des respiratorischen Flusses folgendes umfasst
Berechnen einer Änderung des thorakalen Signals in Bezug auf die Zeit, um eine Ableitung zu erzeugen, die einer zeitlichen Ableitung eines Thoraxvolumens entspricht,
Berechnen einer Änderung des Abdomensignals in Bezug auf die Zeit, um eine weitere Ableitung zu erzeugen, die einer zeitlichen Ableitung eines Abdomensvolumens entspricht, und
der respiratorische Fluss wird durch Kombinieren der Ableitung und der weiteren Ableitung unter Verwendung einer gewichteten Summe bestimmt, die auf dem ersten Kalibrierungskoeffizienten basiert.

6. Verfahren, nach einem der Ansprüche 1 - 5, wobei das Bestimmen des respiratorischen Flusses das Berechnen einer zeitlichen Ableitung einer kalibrierten Summe des thorakalen Signals und des Abdomensignals umfasst, wobei die kalibrierte Summe auf dem ersten Kalibrierungskoeffizienten basiert.

7. Verfahren, nach einem der Ansprüche 1 - 6, wobei die erste Kalibrierung unter Verwendung des thorakalen Signals und des Abdomensignals in Abwesenheit aller respiratorischen Flusssignale bestimmt wird, und/oder
wobei die erste Kalibrierung unter Verwendung des thorakalen Signals und des Abdomensignals in Abwesenheit von respiratorischen Flusssignalen bestimmt wird, die mittels eines oder mehrerer Nasenkanülen gemessen werden, und/oder
wobei die erste Kalibrierung unter Verwendung eines PowerLoss-Kalibrierungsverfahrens durchgeführt wird.

8. Verfahren, nach einem der Ansprüche 1 - 7, wobei die erste Kalibrierung durch Auswählen eines Wertes des Kalibrierungskoeffizienten durchgeführt wird, der eine Funktion minimiert, die ein Verhältnis eines Zählers zu einem Nenner darstellt,
wobei der Zähler eine Potenz einer gewichteten/skalierten Summe des thorakalen Signals und des Abdomensignals ist, wobei eine Gewichtung/Skalierung der gewichteten/skalierten Summe auf dem Wert des Kalibrierungskoeffizienten basiert, und
der Nenner eine Potenz des skalierten thorakalen Signals, aufsummiert mit einer Potenz eines gewichteten/skalierten Abdomensignals ist, welches das Abdomensignal ist, das gewichtet/skaliert wurde, wobei die gewichtete/skalierte Summe auf dem Wert des Kalibrierungskoeffizienten basiert,
wobei ein Gewichtungs-/Skalierungsfaktor, der verwendet wird, um das thorakale Signal relativ zu dem Abdomensignal zu gewichten/skalieren, der Kalibrierungskoeffizient oder eine Funktion des Kalibrierungskoeffizienten ist.

9. Verfahren, nach einem der Ansprüche 1 - 8, wobei die erste Kalibrierung durch Finden des Wertes von k durchgeführt wird, der bis zu einem vorbestimmten Schwellwert die Gleichung ${min}_{k} \frac{RMS \left(\left(1-k\right){dRIP}_{th}+k {dRIP}_{ab}\right)}{RMS \left(\left(1-k\right){dRIP}_{th}\right) + RMS \left(k {dRIP}_{ab}\right)} ,$ erfüllt, wobei RMS der quadratische Mittelwert (root mean square) ist, k ein Testwert des Kalibrierungskoeffizienten ist, dRIPₜₕ eine Ableitung des thorakalen Signals in Bezug auf die Zeit darstellt, dRIP_{ab} eine Ableitung des Abdomensignals in Bezug auf die Zeit darstellt.

10. Verfahren, nach Anspruch 3, wobei die dritte Kalibrierung folgende Schritte umfasst,
Bestimmen eines Korrelationsfaktors zwischen dem thorakalen Signal und dem Abdomensignal, und
Berechnen eines Überschätzungskorrekturfaktors basierend auf dem Korrelationsfaktor, und
wobei das Bestimmen des respiratorischen Flusses ferner umfasst, eine Ableitung einer gewichteten Summe des thorakalen Signals und des Abdomensignals mit dem Überschätzungskorrekturfaktor zu skalieren, und/oder wobei das Bestimmen des respiratorischen Flusses ferner umfasst, das thorakale Signal und das Abdomensignal mit dem Überschätzungskorrekturfaktor zu skalieren.

11. Verfahren, nach einem der Ansprüche 1 - 10, wobei die zweite Kalibrierung folgende Schritte umfasst,
Berechnen eines Nichtlinearitätskorrekturfaktors basierend auf einem Kurvenfit, wobei der Kurvenfit aus Kalibrierungsdaten erzeugt wurde, die den respiratorischen Fluss, der hinsichtlich der Nichtlinearität unkorrigiert ist, und einen Referenzfluss umfassen, der unter Verwendung einer vertrauenswürdigen/validierten Methode gemessen wird und gleichzeitig mit dem respiratorischen Fluss der Kalibrierungsdaten erfasst wird, und
wobei das Bestimmen des respiratorischen Flusses ferner umfasst, eine Ableitung einer gewichteten Summe des thorakalen Signals und des Abdomensignals mit dem Nichtlinearitätskorrekturfaktor zu skalieren, und/oder wobei das Bestimmen des respiratorischen Flusses ferner umfasst, das thorakale Signal und das Abdomensignal mit dem Nichtlinearitätskorrekturfaktor zu skalieren.

12. Verfahren, nach Anspruch 11, wobei der Kurvenfit für das Berechnen des Nichtlinearitätskorrekturfaktors eine Form einer Exponentialfunktion hat und der Nichtlinearitätskorrekturfaktor ein Exponent ist.

13. Computerlesbares Medium, auf dem Anweisungen gespeichert sind, die, wenn sie von einem oder mehreren Prozessoren eines Rechnersystems ausgeführt werden, den einen oder die mehreren Prozessoren veranlassen, die Schritte des Verfahrens nach einem der Ansprüche 1 - 12 auszuführen.

14. Vorrichtung zur Bestimmung eines respiratorischen Flusses aus Daten von Signalen der Respiratorischen Induktionsplethysmographie (RIP), wobei die Vorrichtung folgendes umfasst:
einen Prozessor, der dafür konfiguriert ist,
Daten eines thorakalen Signals eines ersten RIP-Gurts zu empfangen, der in Nähe des Thorax eines Subjekts angeordnet ist;
Daten eines Abdomensignals eines zweiten RIP-Gurts zu empfangen, der in Nähe des Abdomens des Subjekts angeordnet ist; und
einen respiratorischen Fluss des Subjekts auf der Grundlage der Daten des thorakalen Signals und der Daten des Abdomensignals zu bestimmen,
wobei das Bestimmen des respiratorischen Flusses zwei oder mehr Kalibrierungen umfasst, einschließlich:
Durchführen einer ersten Kalibrierung durch Anwenden eines ersten Kalibrierungskoeffizienten, der eine Amplitude einer differentiellen Änderung im thorakalen Signal mit einer Amplitude einer differentiellen Änderung im Abdomensignal in Beziehung setzt, um einen bestimmten respiratorischen Fluss zu erhalten, und
Durchführen einer zweiten Kalibrierung an dem bestimmten respiratorischen Fluss, die eine Nichtlinearität in dem bestimmten respiratorischen Fluss korrigiert.

15. System, umfassend:
eine Vielzahl von Respiratorischen Induktionsplethysmographie-(RIP-)Gurten, einschließlich eines Thoraxgurts, der dafür konfiguriert ist, ein thorakales Signal zu erhalten, und eines Abdomensgurts, der dafür konfiguriert ist, ein Abdomensignal zu erhalten; und
einen Prozessor, der dafür konfiguriert ist,
Daten des thorakalen Signals eines ersten RIP-Gurts zu empfangen, der in Nähe des Thorax eines Subjekts angeordnet ist;
Daten des Abdomensignals eines zweiten RIP-Gurts zu empfangen, der in Nähe des Abdomens des Subjekts angeordnet ist; und
einen respiratorischen Fluss des Subjekts auf der Grundlage der Daten des thorakalen Signals und der Daten des Abdomensignals zu bestimmen,
wobei das Bestimmen des respiratorischen Flusses zwei oder mehr Kalibrierungen umfasst, einschließlich:
Durchführen einer ersten Kalibrierung durch Anwenden eines ersten Kalibrierungskoeffizienten, der eine Amplitude einer differentiellen Änderung im thorakalen Signal mit einer Amplitude einer differentiellen Änderung im Abdomensignal in Beziehung setzt, um einen bestimmten respiratorischen Fluss zu erhalten, und
Durchführen einer zweiten Kalibrierung an dem bestimmten respiratorischen Fluss, die eine Nichtlinearität in dem bestimmten respiratorischen Fluss korrigiert.

## Revendications

1. Procédé mis en œuvre par ordinateur pour déterminer un débit respiratoire à partir de données provenant de signaux de pléthysmographie respiratoire par inductance (RIP), le procédé comprenant :
recevoir des données d'un signal thoracique d'une première ceinture RIP disposée à proximité du thorax d'un sujet ;
recevoir des données d'un signal abdominal d'une seconde ceinture RIP disposée à proximité de l'abdomen du sujet ; et
déterminer un débit respiratoire du sujet sur la base des données du signal thoracique et des données du signal abdominal,
dans lequel la détermination du débit respiratoire comprend au moins deux ou plusieurs étalonnages, comprenant:
effectuer un premier étalonnage par application d'un premier coefficient d'étalonnage qui met en relation une amplitude d'un changement différentiel dans le signal thoracique à une amplitude d'un changement différentiel dans le signal abdominal afin d'obtenir un débit respiratoire déterminé, et
effectuer un deuxième étalonnage sur le débit respiratoire déterminé qui corrige une non-linéarité dans le débit respiratoire déterminé.

2. Procédé selon la revendication 1, comprenant en outre une étape consistant à déterminer un ou plusieurs endotypes d'une apnée obstructive du sommeil ou d'un autre trouble du sommeil du sujet sur la base du débit respiratoire déterminé, étalonné.

3. Procédé selon l'une quelconque des revendications 1 et 2, dans lequel la détermination du débit respiratoire comprend en outre un troisième étalonnage qui corrige une surestimation du débit respiratoire pendant un paradoxe.

4. Procédé selon l'une quelconque des revendications 1 à 3, dans lequel le procédé comprend en outre le calcul de dérivées respectives du signal thoracique et du signal abdominal et la combinaison des dérivées à l'aide d'un coefficient de mise à l'échelle pour déterminer le débit respiratoire.

5. Procédé selon l'une quelconque des revendications 1 à 4, dans lequel la détermination du débit respiratoire comprend :
calculer un changement du signal thoracique en fonction du temps pour générer une dérivée correspondant à une dérivée par rapport au temps d'un volume thoracique ;
calculer un changement du signal abdominal en fonction du temps pour générer une autre dérivée correspondant à une dérivée par rapport au temps d'un volume abdominal, et
le débit respiratoire est déterminé par combinaison de la dérivée et l'autre dérivée à l'aide d'une somme pondérée qui est basée sur le premier coefficient d'étalonnage.

6. Procédé selon l'une quelconque des revendications 1 à 5, dans lequel la détermination du débit respiratoire comprend le calcul d'une dérivée par rapport au temps d'une somme étalonnée du signal thoracique et du signal abdominal, la somme étalonnée étant basée sur le premier coefficient d'étalonnage.

7. Procédé selon l'une quelconque des revendications 1 à 6, dans lequel le premier étalonnage est déterminé à l'aide du signal thoracique et du signal abdominal en l'absence de tous signaux de débit respiratoire, et/ou
dans lequel le premier étalonnage est déterminé à l'aide du signal thoracique et du signal abdominal en l'absence de signaux de débit respiratoire mesurés par une ou plusieurs canules nasales, et/ou
dans lequel le premier étalonnage est effectué à l'aide d'une méthode d'étalonnage PowerLoss.

8. Procédé selon l'une quelconque des revendications 1 à 7, dans lequel le premier étalonnage est effectué par sélection d'une valeur du coefficient d'étalonnage qui rend minimale une fonction qui représente un rapport de numérateur à un dénominateur,
le numérateur étant une puissance d'une somme pondérée/mise à l'échelle du signal thoracique et du signal abdominal, une pondération/mise à l'échelle de la somme pondérée/mise à l'échelle étant basée sur la valeur du coefficient d'étalonnage,
le dénominateur étant une puissance du signal thoracique mis à l'échelle additionnée à une puissance d'un signal abdominal pondéré/mis à l'échelle, qui est le signal abdominal ayant été pondéré/mis à l'échelle sur la base de la valeur du coefficient d'étalonnage,
dans lequel un facteur de pondération/mise à l'échelle utilisé pour pondérer/mettre à l'échelle le signal thoracique par rapport au signal abdominal est le coefficient d'étalonnage ou une fonction du coefficient d'étalonnage.

9. Procédé selon l'une quelconque des revendications 1 à 8, dans lequel on effectue le premier étalonnage en trouvant la valeur de k qui satisfait jusqu'à un seuil prédéterminé l'équation ${min}_{k} \frac{RMS \left(\left(1-k\right){dRIP}_{th}+k {dRIP}_{ab}\right)}{RMS \left(\left(1-k\right){dRIP}_{th}\right) + RMS \left(k {dRIP}_{ab}\right)} ,$ où RMS est la valeur quadratique moyenne, k est une valeur de test du coefficient d'étalonnage, dRIPₜₕ représente une dérivée du signal thoracique par rapport au temps, dRIP_{ab} représente une dérivée du signal abdominal par rapport au temps.

10. Procédé selon la revendication 3, dans lequel le troisième étalonnage comprend les étapes consistant à :
déterminer un facteur de corrélation entre le signal thoracique et le signal abdominal ; et
calculer un facteur de correction de surestimation sur la base du facteur de corrélation, et
dans lequel la détermination du débit respiratoire comprend en outre la mise à l'échelle d'une dérivée d'une somme pondérée du signal thoracique et du signal abdominal par le facteur de correction de surestimation, et/ou dans lequel la détermination du débit respiratoire comprend en outre la mise à l'échelle du signal thoracique et du signal abdominal par le facteur de correction de surestimation.

11. Procédé selon l'une quelconque des revendications 1 à 10, dans lequel le deuxième étalonnage comprend les étapes consistant à :
calculer un facteur de correction de non-linéarité sur la base d'un ajustement de courbe, l'ajustement de courbe ayant été généré à partir de données d'étalonnage qui comprennent le débit respiratoire, qui est non corrigé pour la non-linéarité, et un débit de référence, qui est mesuré à l'aide d'une méthode éprouvée/validée et est acquis en même temps que le débit respiratoire des données d'étalonnage, et
dans lequel la détermination du débit respiratoire comprend en outre la mise à l'échelle d'une dérivée d'une somme pondérée du signal thoracique et du signal abdominal par le facteur de correction de non-linéarité, et/ou dans lequel la détermination du débit respiratoire comprend en outre la mise à l'échelle du signal thoracique et du signal abdominal par le facteur de correction de non-linéarité.

12. Procédé selon la revendication 11, dans lequel l'ajustement de courbe pour le calcul du facteur de correction de non-linéarité présente une forme fonctionnelle exponentielle et le facteur de correction de non-linéarité est un exposant.

13. Support lisible par ordinateur sur lequel sont stockées des instructions qui, lorsqu'elles sont exécutées par un ou plusieurs processeurs d'un système informatique, amènent le ou les processeurs à exécuter les étapes du procédé selon l'une quelconque des revendications 1 à 12.

14. Dispositif pour la détermination d'un débit respiratoire à partir de données provenant de signaux de pléthysmographie respiratoire par inductance (RIP), le dispositif comprenant :
un processeur configuré pour
recevoir des données d'un signal thoracique d'une première ceinture RIP disposée à proximité du thorax d'un sujet ;
recevoir des données d'un signal abdominal d'une seconde ceinture RIP disposée à proximité de l'abdomen du sujet ; et
déterminer un débit respiratoire du sujet sur la base des données du signal thoracique et des données du signal abdominal,
dans lequel la détermination du débit respiratoire comprend au moins deux étalonnages, comprenant :
effectuer un premier étalonnage par application d'un premier coefficient d'étalonnage qui met en relation une amplitude d'un changement différentiel dans le signal thoracique à une amplitude d'un changement différentiel dans le signal abdominal pour obtenir un débit respiratoire déterminé, et
effectuer un deuxième étalonnage sur le débit respiratoire déterminé qui corrige une non-linéarité dans le débit respiratoire déterminé.

15. Système comprenant :
une pluralité de ceintures pour pléthysmographie respiratoire par inductance (RIP), comprenant une ceinture thoracique configurée pour obtenir un signal thoracique et une ceinture abdominale configurée pour obtenir un signal abdominal ; et
un processeur configuré pour
recevoir des données du signal thoracique d'une première ceinture RIP disposée à proximité du thorax d'un sujet ;
recevoir des données du signal abdominal d'une seconde ceinture RIP disposée à proximité de l'abdomen du sujet ; et
déterminer un débit respiratoire du sujet sur la base des données du signal thoracique et des données du signal abdominal,
dans lequel la détermination du débit respiratoire comprend au moins deux étalonnages, comprenant :
effectuer un premier étalonnage par application d'un premier coefficient d'étalonnage qui met en relation une amplitude d'un changement différentiel dans le signal thoracique à une amplitude d'un changement différentiel dans le signal abdominal pour obtenir un débit respiratoire déterminé, et
effectuer un deuxième étalonnage sur le débit respiratoire déterminé qui corrige une non-linéarité dans le débit respiratoire déterminé.
